# EUROPEAN PATENT SPECIFICATION

(11) **EP 3 008 186 B1**
(45) Date of publication and mention of the grant of the patent: **28.11.2018**
(21) Application number: 14732023.8
(22) Date of filing: 13.06.2014
(51) Int. Cl.: C12N 15/82

(54) **METHODS FOR NON-TRANSGENIC GENOME EDITING IN PLANTS**
VERFAHREN ZUR NICHTTRANSGENEN GENOMÄNDERUNG BEI PFLANZEN
PROCÉDÉS D'ÉDITION DE GÉNOME NON TRANSGÉNIQUE DANS DES PLANTES

(30) Priority: 14.06.2013 US 201361835307 P
(43) Date of publication of application: 20.04.2016
(73) Proprietor: Cellectis, 75013 Paris (FR)
(72) Inventor: VOYTAS, Daniel, Falcon Heights, Minnesota 55108 (US); ZHANG, Feng, Maple Grove, MN 55311 (US); LI, Jin, Shoreview, Minnesota 55126 (US); STODDARD, Thomas, St. Louis Park, Minnesota 55426 (US); LUO, Song, Chicago, Illinois 60615 (US)
(74) Representative: D Young & Co LLP
(86) International application number: PCT/IB2014/062223
(87) International publication number: WO 2014/199358

(56) References cited:
- WO-A1-2008/148223
- WO-A1-2013/009525
- WO-A2-2012/129443
- WO-A2-2014/186585
- WO-A2-2014/191521
- VOYTAS DANIEL F: "Plant genome engineering with sequence-specific nucleases.", ANNUAL REVIEW OF PLANT BIOLOGY 2013, vol. 64, 2013, pages 327-350, XP002729239, ISSN: 1545-2123
- ZHANG YONG ET AL: "Transcription Activator-Like Effector Nucleases Enable Efficient Plant Genome Engineering", PLANT PHYSIOLOGY (ROCKVILLE), vol. 161, no. 1, January 2013 (2013-01), pages 20-27, XP55070911, ISSN: 0032-0889
- WEI CHUANXIAN ET AL: "TALEN or Cas9-Rapid, Efficient and Specific Choices for Genome Modifications", JOURNAL OF GENETICS AND GENOMICS, vol. 40, no. 6, June 2013 (2013-06), pages 281-289, XP002729241,
- WANG C-Y ET AL: "IMPROVED CYTOPLASMIC DELIVERY TO PLANT PROTOPLASTS VIA PH-SENSITIVE LIPOSOMES", PLANT PHYSIOLOGY (ROCKVILLE), vol. 82, no. 1, 1986, pages 179-184, XP002729242, ISSN: 0032-0889

### SEQUENCE LISTING

<110> CELLECTIS
<120> METHODS FOR NON-TRANSGENIC GENOME EDITING IN PLANTS
<130> 36861-0005WO1
<150> 61/835,307
   <151> 2013-06-14
<160> 55
<170> PatentIn version 3.5
<210> 1
   <211> 720
   <212> DNA
   <213> artficial sequence
<220>
   <223> I-sceI coding sequence
<400> 1
   atggccaaaa acatcaaaaa aaaccaggta atgaacctgg gtccgaactc taaactgctg      60
   aaagaataca aatcccagct gatcgaactg aacatcgaac agttcgaagc aggtatcggt     120
   ctgatcctgg gtgatgctta catccgttct cgtgatgaag gtaaaaccta ctgtatgcag     180
   ttcgagtgga aaaacaaagc atacatggac cacgtatgtc tgctgtacga tcagtgggta     240
   ctgtccccgc cgcacaaaaa agaacgtgtt aaccacctgg gtaacctggt aatcacctgg     300
   ggcgcccaga ctttcaaaca ccaagctttc aacaaactgg ctaacctgtt catcgttaac     360
   aacaaaaaaa ccatcccgaa caacctggtt gaaaactacc tgaccccgat gtctctggca     420
   tactggttca tggatgatgg tggtaaatgg gattacaaca aaaactctac caacaaatcg     480
   atcgtactga acacccagtc tttcactttc gaagaagtag aatacctggt taagggtctg     540
   cgtaacaaat tccaactgaa ctgttacgta aaaatcaaca aaaacaaacc gatcatctac     600
   atcgattcta tgtcttacct gatcttctac aacctgatca aaccgtacct gatcccgcag     660
   atgatgtaca aactgccgaa cactatctcc tccgaaactt tcctgaaagc ggccgactaa     720
<210> 2
   <211> 2814
   <212> DNA
   <213> artficial sequence
<220>
   <223> TAL domain ALS2T1_L coding sequence
<400> 2
   atgggcgatc ctaaaaagaa acgtaaggtc atcgattacc catacgatgt tccagattac      60
   gctatcgata tcgccgatct acgcacgctc ggctacagcc agcagcaaca ggagaagatc     120
   aaaccgaagg ttcgttcgac agtggcgcag caccacgagg cactggtcgg ccacgggttt     180
   acacacgcgc acatcgttgc gttaagccaa cacccggcag cgttagggac cgtcgctgtc     240
   aagtatcagg acatgatcgc agcgttgcca gaggcgacac acgaagcgat cgttggcgtc     300
   ggcaaacagt ggtccggcgc acgcgctctg gaggccttgc tcacggtggc gggagagttg     360
   agaggtccac cgttacagtt ggacacaggc caacttctca agattgcaaa acgtggcggc     420
   gtgaccgcag tggaggcagt gcatgcatgg cgcaatgcac tgacgggtgc cccgctcaac     480
   ttgaccccgg agcaggtggt ggccatcgcc agcaatattg gtggcaagca ggcgctggag     540
   acggtgcagg cgctgttgcc ggtgctgtgc caggcccacg gcttgacccc ccagcaggtg     600
   gtggccatcg ccagcaataa tggtggcaag caggcgctgg agacggtcca gcggctgttg     660
   ccggtgctgt gccaggccca cggcttgacc ccggagcagg tggtggccat cgccagccac     720
   gatggcggca agcaggcgct ggagacggtc cagcggctgt tgccggtgct gtgccaggcc     780
   cacggcttga ccccccagca ggtggtggcc atcgccagca atggcggtgg caagcaggcg     840
   ctggagacgg tccagcggct gttgccggtg ctgtgccagg cccacggctt gaccccccag    900
   caggtggtgg ccatcgccag caatggcggt ggcaagcagg cgctggagac ggtccagcgg     960
   ctgttgccgg tgctgtgcca ggcccacggc ttgacccccc agcaggtggt ggccatcgcc    1020
   agcaataatg gtggcaagca ggcgctggag acggtccagc ggctgttgcc ggtgctgtgc    1080
   caggcccacg gcttgacccc ccagcaggtg gtggccatcg ccagcaatgg cggtggcaag    1140
   caggcgctgg agacggtcca gcggctgttg ccggtgctgt gccaggccca cggcttgacc    1200
   ccccagcagg tggtggccat cgccagcaat ggcggtggca agcaggcgct ggagacggtc    1260
   cagcggctgt tgccggtgct gtgccaggcc cacggcttga ccccggagca ggtggtggcc    1320
   atcgccagcc acgatggcgg caagcaggcg ctggagacgg tccagcggct gttgccggtg    1380
   ctgtgccagg cccacggctt gaccccggag caggtggtgg ccatcgccag ccacgatggc    1440
   ggcaagcagg cgctggagac ggtccagcgg ctgttgccgg tgctgtgcca ggcccacggc    1500
   ttgaccccgg agcaggtggt ggccatcgcc agcaatattg gtggcaagca ggcgctggag    1560
   acggtgcagg cgctgttgcc ggtgctgtgc caggcccacg gcttgacccc ggagcaggtg    1620
   gtggccatcg ccagccacga tggcggcaag caggcgctgg agacggtcca gcggctgttg    1680
   ccggtgctgt gccaggccca cggcttgacc ccggagcagg tggtggccat cgccagcaat    1740
   attggtggca agcaggcgct ggagacggtg caggcgctgt tgccggtgct gtgccaggcc    1800
   cacggcttga ccccccagca ggtggtggcc atcgccagca atggcggtgg caagcaggcg    1860
   ctggagacgg tccagcggct gttgccggtg ctgtgccagg cccacggctt gaccccccag   1920
   caggtggtgg ccatcgccag caatggcggt ggcaagcagg cgctggagac ggtccagcgg    1980
   ctgttgccgg tgctgtgcca ggcccacggc ttgacccctc agcaggtggt ggccatcgcc    2040
   agcaatggcg gcggcaggcc ggcgctggag agcattgttg cccagttatc tcgccctgat    2100
   ccggcgttgg ccgcgttgac caacgaccac ctcgtcgcct tggcctgcct cggcgggcgt    2160
   cctgcgctgg atgcagtgaa aaagggattg gggatcccta tcagccgttc ccagctggtg    2220
   aagtccgagc tggaggagaa gaaatccgag ttgaggcaca agctgaagta cgtgccccac    2280
   gagtacatcg agctgatcga gatcgcccgg aacagcaccc aggaccgtat cctggagatg    2340
   aaggtgatgg agttcttcat gaaggtgtac ggctacaggg gcaagcacct gggcggctcc    2400
   aggaagcccg acggcgccat ctacaccgtg ggctccccca tcgactacgg cgtgatcgtg    2460
   gacaccaagg cctactccgg cggctacaac ctgcccatcg gccaggccga cgaaatgcag    2520
   aggtacgtgg aggagaacca gaccaggaac aagcacatca accccaacga gtggtggaag    2580
   gtgtacccct ccagcgtgac cgagttcaag ttcctgttcg tgtccggcca cttcaagggc    2640
   aactacaagg cccagctgac caggctgaac cacatcacca actgcaacgg cgccgtgctg    2700
   tccgtggagg agctcctgat cggcggcgag atgatcaagg ccggcaccct gaccctggag    2760
   gaggtgagga ggaagttcaa caacggcgag atcaacttcg cggccgactg ataa          2814
<210> 3
   <211> 2832
   <212> DNA
   <213> artficial sequence
<220>
   <223> TAL domain targeting ALS2T1_R coding sequence
<400> 3
   atgggcgatc ctaaaaagaa acgtaaggtc atcgataagg agaccgccgc tgccaagttc     60
   gagagacagc acatggacag catcgatatc gccgatctac gcacgctcgg ctacagccag    120
   cagcaacagg agaagatcaa accgaaggtt cgttcgacag tggcgcagca ccacgaggca    180
   ctggtcggcc acgggtttac acacgcgcac atcgttgcgt taagccaaca cccggcagcg    240
   ttagggaccg tcgctgtcaa gtatcaggac atgatcgcag cgttgccaga ggcgacacac    300
   gaagcgatcg ttggcgtcgg caaacagtgg tccggcgcac gcgctctgga ggccttgctc    360
   acggtggcgg gagagttgag aggtccaccg ttacagttgg acacaggcca acttctcaag    420
   attgcaaaac gtggcggcgt gaccgcagtg gaggcagtgc atgcatggcg caatgcactg    480
   acgggtgccc cgctcaactt gaccccggag caggtggtgg ccatcgccag ccacgatggc    540
   ggcaagcagg cgctggagac ggtccagcgg ctgttgccgg tgctgtgcca ggcccacggc    600
   ttgacccccc agcaggtggt ggccatcgcc agcaatggcg gtggcaagca ggcgctggag    660
   acggtccagc ggctgttgcc ggtgctgtgc caggcccacg gcttgacccc ccagcaggtg    720
   gtggccatcg ccagcaataa tggtggcaag caggcgctgg agacggtcca gcggctgttg    780
   ccggtgctgt gccaggccca cggcttgacc ccggagcagg tggtggccat cgccagcaat    840
   attggtggca agcaggcgct ggagacggtg caggcgctgt tgccggtgct gtgccaggcc    900
   cacggcttga ccccggagca ggtggtggcc atcgccagcc acgatggcgg caagcaggcg    960
   ctggagacgg tccagcggct gttgccggtg ctgtgccagg cccacggctt gaccccggag   1020
   caggtggtgg ccatcgccag ccacgatggc ggcaagcagg cgctggagac ggtccagcgg   1080
   ctgttgccgg tgctgtgcca ggcccacggc ttgaccccgg agcaggtggt ggccatcgcc   1140
   agccacgatg gcggcaagca ggcgctggag acggtccagc ggctgttgcc ggtgctgtgc   1200
   caggcccacg gcttgacccc ggagcaggtg gtggccatcg ccagcaatat tggtggcaag   1260
   caggcgctgg agacggtgca ggcgctgttg ccggtgctgt gccaggccca cggcttgacc   1320
   ccccagcagg tggtggccat cgccagcaat aatggtggca agcaggcgct ggagacggtc   1380
   cagcggctgt tgccggtgct gtgccaggcc cacggcttga ccccggagca ggtggtggcc   1440
   atcgccagcc acgatggcgg caagcaggcg ctggagacgg tccagcggct gttgccggtg   1500
   ctgtgccagg cccacggctt gaccccggag caggtggtgg ccatcgccag caatattggt   1560
   ggcaagcagg cgctggagac ggtgcaggcg ctgttgccgg tgctgtgcca ggcccacggc   1620
   ttgacccccc agcaggtggt ggccatcgcc agcaatggcg gtggcaagca ggcgctggag   1680
   acggtccagc ggctgttgcc ggtgctgtgc caggcccacg gcttgacccc ccagcaggtg   1740
   gtggccatcg ccagcaataa tggtggcaag caggcgctgg agacggtcca gcggctgttg   1800
   ccggtgctgt gccaggccca cggcttgacc ccggagcagg tggtggccat cgccagcaat   1860
   attggtggca agcaggcgct ggagacggtg caggcgctgt tgccggtgct gtgccaggcc   1920
   cacggcttga ccccggagca ggtggtggcc atcgccagcc acgatggcgg caagcaggcg   1980
   ctggagacgg tccagcggct gttgccggtg ctgtgccagg cccacggctt gacccctcag   2040
   caggtggtgg ccatcgccag caatggcggc ggcaggccgg cgctggagag cattgttgcc   2100
   cagttatctc gccctgatcc ggcgttggcc gcgttgacca acgaccacct cgtcgccttg   2160
   gcctgcctcg gcgggcgtcc tgcgctggat gcagtgaaaa agggattggg ggatcctatc   2220
   agccgttccc agctggtgaa gtccgagctg gaggagaaga aatccgagtt gaggcacaag   2280
   ctgaagtacg tgccccacga gtacatcgag ctgatcgaga tcgcccggaa cagcacccag   2340
   gaccgtatcc tggagatgaa ggtgatggag ttcttcatga aggtgtacgg ctacaggggc   2400
   aagcacctgg gcggctccag gaagcccgac ggcgccatct acaccgtggg ctcccccatc   2460
   gactacggcg tgatcgtgga caccaaggcc tactccggcg gctacaacct gcccatcggc   2520
   caggccgacg aaatgcagag gtacgtggag gagaaccaga ccaggaacaa gcacatcaac   2580
   cccaacgagt ggtggaaggt gtacccctcc agcgtgaccg agttcaagtt cctgttcgtg   2640
   tccggccact tcaagggcaa ctacaaggcc cagctgacca ggctgaacca catcaccaac   2700
   tgcaacggcg ccgtgctgtc cgtggaggag ctcctgatcg gcggcgagat gatcaaggcc   2760
   ggcaccctga ccctggagga ggtgaggagg aagttcaaca acggcgagat caacttcgcg   2820
   gccgactgat aa                                                       2832
<210> 4
   <211> 2810
   <212> DNA
   <213> artficial sequence
<220>
   <223> TAL domain targeting Xyl_T04_L1 coding sequence
<400> 4
   atgggcgatc ctaaaaagaa acgtaaggtc atcgattacc catacgatgt tccagattac      60
   gctatcgata tcgccgatct acgcacgctc ggctacagcc agcagcaaca ggagaagatc     120
   aaaccgaagg ttcgttcgac agtggcgcag caccacgagg cactggtcgg ccacgggttt     180
   acacacgcgc acatcgttgc gttaagccaa cacccggcag cgttagggac cgtcgctgtc     240
   aagtatcagg acatgatcgc agcgttgcca gaggcgacac acgaagcgat cgttggcgtc     300
   ggcaaacagt ggtccggcgc acgcgctctg gaggccttgc tcacggtggc gggagagttg     360
   agaggtccac cgttacagtt ggacacaggc caacttctca agattgcaaa acgtggcggc     420
   gtgaccgcag tggaggcagt gcatgcatgg cgcaatgcac tgacgggtgc cccgctcaac     480
   ttgaccccgg agcaggtggt ggccatcgcc agccacgatg gcggcaagca ggcgctggag     540
   acggtccagc ggctgttgcc ggtgctgtgc caggcccacg gcttgacccc ccagcaggtg     600
   gtggccatcg ccagcaatgg cggtggcaag caggcgctgg agacggtcca gcggctgttg     660
   ccggtgctgt gccaggccca cggcttgacc ccggagcagg tggtggccat cgccagccac     720
   gatggcggca agcaggcgct ggagacggtc cagcggctgt tgccggtgct gtgccaggcc     780
   cacggcttga ccccccagca ggtggtggcc atcgccagca atggcggtgg caagcaggcg     840
   ctggagacgg tccagcggct gttgccggtg ctgtgccagg cccacggctt gaccccccag     900
   caggtggtgg ccatcgccag caatggcggt ggcaagcagg cgctggagac ggtccagcgg     960
   ctgttgccgg tgctgtgcca ggcccacggc ttgaccccgg agcaggtggt ggccatcgcc    1020
   agccacgatg gcggcaagca ggcgctggag acggtccagc ggctgttgcc ggtgctgtgc    1080
   caggcccacg gcttgacccc ccagcaggtg gtggccatcg ccagcaataa tggtggcaag    1140
   caggcgctgg agacggtcca gcggctgttg ccggtgctgt gccaggccca cggcttgacc    1200
   cggagcaggt ggtggccatc gccagccacg atggcggcaa gcaggcgctg gagacggtcc    1260
   agcggctgtt gccggtgctg tgccaggccc acggcttgac cccccagcag gtggtggcca    1320
   tcgccagcaa tggcggtggc aagcaggcgc tggagacggt ccagcggctg ttgccggtgc    1380
   tgtgccaggc ccacggcttg accccggagc aggtggtggc catcgccagc cacgatggcg    1440
   gcaagcaggc gctggagacg gtccagcggc tgttgccggt gctgtgccag gcccacggct    1500
   tgacccccca gcaggtggtg gccatcgcca gcaatggcgg tggcaagcag gcgctggaga    1560
   cggtccagcg gctgttgccg gtgctgtgcc aggcccacgg cttgaccccg gagcaggtgg    1620
   tggccatcgc cagccacgat ggcggcaagc aggcgctgga gacggtccag cggctgttgc    1680
   cggtgctgtg ccaggcccac ggcttgaccc cggagcaggt ggtggccatc gccagcaata    1740
   ttggtggcaa gcaggcgctg gagacggtgc aggcgctgtt gccggtgctg tgccaggccc    1800
   acggcttgac cccggagcag gtggtggcca tcgccagcaa tattggtggc aagcaggcgc    1860
   tggagacggt gcaggcgctg ttgccggtgc tgtgccaggc ccacggcttg accccggagc    1920
   aggtggtggc catcgccagc cacgatggcg gcaagcaggc gctggagacg gtccagcggc    1980
   tgttgccggt gctgtgccag gcccacggct tgacccctca gcaggtggtg gccatcgcca    2040
   gcaatggcgg cggcaggccg gcgctggaga gcattgttgc ccagttatct cgccctgatc    2100
   cggcgttggc cgcgttgacc aacgaccacc tcgtcgcctt ggcctgcctc ggcgggcgtc    2160
   ctgcgctgga tgcagtgaaa aagggattgg gggatcctat cagccgttcc cagctggtga    2220
   agtccgagct ggaggagaag aaatccgagt tgaggcacaa gctgaagtac gtgccccacg    2280
   agtacatcga gctgatcgag atcgcccgga acagcaccca ggaccgtatc ctggagatga    2340
   aggtgatgga gttcttcatg aaggtgtacg gctacagggg caagcacctg ggcggctcca    2400
   ggaagcccga cggcgccatc tacaccgtgg gctcccccat cgactacggc gtgatcgtgg    2460
   acaccaaggc ctactccggc ggctacaacc tgcccatcgg ccaggccgac gaaatgcaga    2520
   ggtacgtgga ggagaaccag accaggaaca agcacatcaa ccccaacgag tggtggaagg    2580
   tgtacccctc cagcgtgacc gagttcaagt tcctgttcgt gtccggccac ttcaagggca    2640
   actacaaggc ccagctgacc aggctgaacc acatcaccaa ctgcaacggc gccgtgctgt    2700
   ccgtggagga gctcctgatc ggcggcgaga tgatcaaggc cggcaccctg accctggagg    2760
   aggtgaggag gaagttcaac aacggcgaga tcaacttcgc ggccgactga               2810
<210> 5
   <211> 2829
   <212> DNA
   <213> artficial sequence
<220>
   <223> TAL domain targeting Xyl_T04_R1 coding sequence
<400> 5
   atgggcgatc ctaaaaagaa acgtaaggtc atcgataagg agaccgccgc tgccaagttc     60
   gagagacagc acatggacag catcgatatc gccgatctac gcacgctcgg ctacagccag    120
   cagcaacagg agaagatcaa accgaaggtt cgttcgacag tggcgcagca ccacgaggca    180
   ctggtcggcc acgggtttac acacgcgcac atcgttgcgt taagccaaca cccggcagcg    240
   ttagggaccg tcgctgtcaa gtatcaggac atgatcgcag cgttgccaga ggcgacacac    300
   gaagcgatcg ttggcgtcgg caaacagtgg tccggcgcac gcgctctgga ggccttgctc    360
   acggtggcgg gagagttgag aggtccaccg ttacagttgg acacaggcca acttctcaag    420
   attgcaaaac gtggcggcgt gaccgcagtg gaggcagtgc atgcatggcg caatgcactg    480
   acgggtgccc cgctcaactt gaccccccag caggtggtgg ccatcgccag caataatggt    540
   ggcaagcagg cgctggagac ggtccagcgg ctgttgccgg tgctgtgcca ggcccacggc    600
   ttgacccccc agcaggtggt ggccatcgcc agcaataatg gtggcaagca ggcgctggag    660
   acggtccagc ggctgttgcc ggtgctgtgc caggcccacg gcttgacccc ccagcaggtg    720
   gtggccatcg ccagcaataa tggtggcaag caggcgctgg agacggtcca gcggctgttg    780
   ccggtgctgt gccaggccca cggcttgacc ccggagcagg tggtggccat cgccagcaat    840
   attggtggca agcaggcgct ggagacggtg caggcgctgt tgccggtgct gtgccaggcc    900
   cacggcttga ccccggagca ggtggtggcc atcgccagca atattggtgg caagcaggcg    960
   ctggagacgg tgcaggcgct gttgccggtg ctgtgccagg cccacggctt gaccccccag   1020
   caggtggtgg ccatcgccag caataatggt ggcaagcagg cgctggagac ggtccagcgg   1080
   ctgttgccgg tgctgtgcca ggcccacggc ttgaccccgg agcaggtggt ggccatcgcc   1140
   agcaatattg gtggcaagca ggcgctggag acggtgcagg cgctgttgcc ggtgctgtgc   1200
   caggcccacg gcttgacccc ccagcaggtg gtggccatcg ccagcaataa tggtggcaag   1260
   caggcgctgg agacggtcca gcggctgttg ccggtgctgt gccaggccca cggcttgacc   1320
   ccggagcagg tggtggccat cgccagcaat attggtggca agcaggcgct ggagacggtg   1380
   caggcgctgt tgccggtgct gtgccaggcc cacggcttga ccccggagca ggtggtggcc   1440
   atcgccagca atattggtgg caagcaggcg ctggagacgg tgcaggcgct gttgccggtg   1500
   ctgtgccagg cccacggctt gaccccccag caggtggtgg ccatcgccag caataatggt   1560
   ggcaagcagg cgctggagac ggtccagcgg ctgttgccgg tgctgtgcca ggcccacggc   1620
   ttgacccccc agcaggtggt ggccatcgcc agcaatggcg gtggcaagca ggcgctggag   1680
   acggtccagc ggctgttgcc ggtgctgtgc caggcccacg gcttgacccc ggagcaggtg   1740
   gtggccatcg ccagcaatat tggtggcaag caggcgctgg agacggtgca ggcgctgttg   1800
   ccggtgctgt gccaggccca cggcttgacc ccccagcagg tggtggccat cgccagcaat   1860
   aatggtggca agcaggcgct ggagacggtc cagcggctgt tgccggtgct gtgccaggcc   1920
   cacggcttga ccccggagca ggtggtggcc atcgccagca atattggtgg caagcaggcg   1980
   ctggagacgg tgcaggcgct gttgccggtg ctgtgccagg cccacggctt gacccctcag   2040
   caggtggtgg ccatcgccag caatggcggc ggcaggccgg cgctggagag cattgttgcc   2100
   cagttatctc gccctgatcc ggcgttggcc gcgttgacca acgaccacct cgtcgccttg   2160
   gcctgcctcg gcgggcgtcc tgcgctggat gcagtgaaaa agggattggg ggatcctatc   2220
   agccgttccc agctggtgaa gtccgagctg gaggagaaga aatccgagtt gaggcacaag   2280
   ctgaagtacg tgccccacga gtacatcgag ctgatcgaga tcgcccggaa cagcacccag   2340
   gaccgtatcc tggagatgaa ggtgatggag ttcttcatga aggtgtacgg ctacaggggc   2400
   aagcacctgg gcggctccag gaagcccgac ggcgccatct acaccgtggg ctcccccatc   2460
   gactacggcg tgatcgtgga caccaaggcc tactccggcg gctacaacct gcccatcggc   2520
   caggccgacg aaatgcagag gtacgtggag gagaaccaga ccaggaacaa gcacatcaac   2580
   cccaacgagt ggtggaaggt gtacccctcc agcgtgaccg agttcaagtt cctgttcgtg   2640
   tccggccact tcaagggcaa ctacaaggcc cagctgacca ggctgaacca catcaccaac   2700
   tgcaacggcg ccgtgctgtc cgtggaggag ctcctgatcg gcggcgagat gatcaaggcc   2760
   ggcaccctga ccctggagga ggtgaggagg aagttcaaca acggcgagat caacttcgcg   2820
   gccgactga                                                          2829
<210> 6
   <211> 2262
   <212> DNA
   <213> homo sapiens
<220>
   <223> Trex2 polynucleotide sequence
<400> 6
   atgggcgggg cgcggctcgg agcgcgaaac atggcggggc aggacgctgg ctgcggccgt     60
   ggcggcgacg actactcaga ggacgaaggc gacagcagcg tgtccagggc ggctgtggag    120
   gtgttcggga agctgaagga cctaaactgc cccttcctcg agggtctgta tatcacagag    180
   ccaaagacaa ttcaggaact gctgtgcagc ccctcagagt accgcttgga gatcctagag    240
   tggatgtgta cccgggtctg gccctcactg caggacaggt tcagctcact gaaaggggtc    300
   ccaacagagg tgaagatcca agaaatgacg aagctgggcc acgagctgat gctgtgtgcg    360
   ccagatgacc aggagctcct caagggctgt gcctgcgccc agaagcagct acacttcatg    420
   gaccagttgc tcgataccat ccggagcctg accattgggt gctccagttg ctcgagcctg    480
   atggagcact tcgaggacac cagggagaag aacgaggcct tgctggggga gctcttctct    540
   agcccccacc tgcagatgct cctgaatcca gagtgcgacc cgtggcccct ggacatgcag    600
   cccctcctca acaagcagag tgatgactgg cagtgggcca gtgcctctgc caagtccgag    660
   gaggaggaga agctggcgga gcttgccagg cagctgcagg agagtgctgc caagttgcac    720
   gcgcttagaa cggagtactt tgcacagcat gagcaagggg ctgctgcggg cgcagccgac    780
   atcagcaccc tagaccagaa gctgcgtctg gtcacttccg acttccacca gctaatcttg    840
   gcttttctcc aagtctacga cgacgagctg ggcgagtgct gccagcgccc aggccctgac    900
   ctccacccgt gcggccccat catccaggcc acgcaccaga atctgacttc ctacagccaa    960
   atccccagag gccaacctaa aaagccggct ttagttacga tgactacagt tcccacgtgc   1020
   gcaactctgc ccttggctca aggattccgt gatgttcatt ttggttttct aagcgagagg   1080
   ctccgagcct tccaacctct gactggctgg tcctgtgaga cccctcgatc agggatgctg   1140
   ctgcaagtgg tcatggcagt tgctgacacc tctgcgaagg ccgtggagac cgtgaagaag   1200
   cagcaaggcg agcagatctg ctggggtggc agcagctccg tcatgagtct agctaccaag   1260
   atgaatgaac taatggagaa atagaaagtc ttcagtgatg gcctacgcca aagcacagga   1320
   tggggcgggc aggaagccct ctcccaagat cgagttggcc gaggatggat gattgtggca   1380
   gcagaagccg ttgcagcccc acgttgtgct ctaggcagct gggggcgggc tgcggccgct   1440
   gattaaaggc cgcctagagc agcctgtgtg gcgacaggtg cccagaagcc caggaagccg   1500
   gtcagtgccc gccccagttt gaggacttgc tatccccgtg ggaacatcac catgtccgag   1560
   gcaccccggg ccgagacctt tgtcttcctg gacctggaag ccactgggct ccccagtgtg   1620
   gagcccgaga ttgccgagct gtccctcttt gctgtccacc gctcctccct ggagaacccg   1680
   gagcacgacg agtctggtgc cctagtattg ccccgggtcc tggacaagct cacgctgtgc   1740
   atgtgcccgg agcgcccctt cactgccaag gccagcgaga tcaccggcct gagcagtgag   1800
   ggcctggcgc gatgccggaa ggctggcttt gatggcgccg tggtgcggac gctgcaggcc   1860
   ttcctgagcc gccaggcagg gcccatctgc cttgtggccc acaatggctt tgattatgat   1920
   ttccccctgc tgtgtgccga gctgcggcgc ctgggtgccc gcctgccccg ggacactgtc   1980
   tgcctggaca cgctgccggc cctgcggggc ctggaccgcg cccacagcca cggcacccgg   2040
   gcccggggcc gccagggtta cagcctcggc agcctcttcc accgctactt ccgggcagag   2100
   ccaagcgcag cccactcagc cgagggcgac gtgcacaccc tgctcctgat cttcctgcac   2160
   cgcgccgcag agctgctcgc ctgggccgat gagcaggccc gtgggtgggc ccacatcgag   2220
   cccatgtact tgccgcctga tgaccccagc ctggaggcct ga                      2262
<210> 7
   <211> 1458
   <212> DNA
   <213> artficial sequence
<220>
   <223> single chain Trex2 coding sequence
<400> 7
   atgggttccg aggcaccccg ggccgagacc tttgtcttcc tggacctgga agccactggg    60
   ctccccagtg tggagcccga gattgccgag ctgtccctct ttgctgtcca ccgctcctcc   120
   ctggagaacc cggagcacga cgagtctggt gccctagtat tgccccgggt cctggacaag   180
   ctcacgctgt gcatgtgccc ggagcgcccc ttcactgcca aggccagcga gatcaccggc   240
   ctgagcagtg agggcctggc gcgatgccgg aaggctggct ttgatggcgc cgtggtgcgg   300
   acgctgcagg ccttcctgag ccgccaggca gggcccatct gccttgtggc ccacaatggc   360
   tttgattatg atttccccct gctgtgtgcc gagctgcggc gcctgggtgc ccgcctgccc   420
   cgggacactg tctgcctgga cacgctgccg gccctgcggg gcctggaccg cgcccacagc   480
   cacggcaccc gggcccgggg ccgccagggt tacagcctcg gcagcctctt ccaccgctac   540
   ttccgggcag agccaagcgc agcccactca gccgagggcg acgtgcacac cctgctcctg   600
   atcttcctgc accgcgccgc agagctgctc gcctgggccg atgagcaggc ccgtgggtgg   660
   gcccacatcg agcccatgta cttgccgcct gatgacccca gcctggaggc gactcctcca   720
   cagaccggtc tggatgttcc ttactccgag gcaccccggg ccgagacctt tgtcttcctg   780
   gacctggaag ccactgggct ccccagtgtg gagcccgaga ttgccgagct gtccctcttt   840
   gctgtccacc gctcctccct ggagaacccg gagcacgacg agtctggtgc cctagtattg   900
   ccccgggtcc tggacaagct cacgctgtgc atgtgcccgg agcgcccctt cactgccaag   960
   gccagcgaga tcaccggcct gagcagtgag ggcctggcgc gatgccggaa ggctggcttt  1020
   gatggcgccg tggtgcggac gctgcaggcc ttcctgagcc gccaggcagg gcccatctgc  1080
   cttgtggccc acaatggctt tgattatgat ttccccctgc tgtgtgccga gctgcggcgc  1140
   ctgggtgccc gcctgccccg ggacactgtc tgcctggaca cgctgccggc cctgcggggc  1200
   ctggaccgcg cccacagcca cggcacccgg gcccggggcc gccagggtta cagcctcggc  1260
   agcctcttcc accgctactt ccgggcagag ccaagcgcag cccactcagc cgagggcgac  1320
   gtgcacaccc tgctcctgat cttcctgcac cgcgccgcag agctgctcgc ctgggccgat  1380
   gagcaggccc gtgggtgggc ccacatcgag cccatgtact tgccgcctga tgaccccagc  1440
   ctggaggcgg ccgactga                                                1458
<210> 8
   <211> 85
   <212> DNA
   <213> Nicotiana tabaccum
<400> 8
   gatcgcagat ccccgggtac ccgggatcct gcagtcgacg ctagggataa cagggtaata 60
   cagattcgag cccaattcat aaatt                                       85
<210> 9
   <211> 83
   <212> DNA
   <213> Artificial Sequence
<220>
   <223> synthetic nucleic acid
<400> 9
   gatcgcagat ccccgggtac ccgggagcct gcagtcgacg ctagggaaca gggtaataca 60
   gattcgagcc caattcataa att                                         83
<210> 10
   <211> 81
   <212> DNA
   <213> Artificial Sequence
<220>
   <223> synthetic nucleic acid
<400> 10
   gatcgcagat ccccgggtac ccgggatcct gcagtcgacg ctagggcagg gtaatacaga 60
   ttcgagccca attcataaat t                                           81
<210> 11
   <211> 80
   <212> DNA
   <213> Artificial Sequence
<220>
   <223> synthetic nucleic acid
<400> 11
   gatcgcagat ccccgggtac ccgggagcct gcagtcgacg ctagggaggg taatacagat 60
   tcgagcccaa ttcataaatt                                             80
<210> 12
   <211> 78
   <212> DNA
   <213> Artificial Sequence
<220>
   <223> synthetic nucleic acid
<400> 12
   gatcgcagat ccccgggtac ccgggatcct gcagtcgacg ctacagggta atacagattc 60
   gagcccaatt cataaatt                                               78
<210> 13
   <211> 76
   <212> DNA
   <213> Artificial Sequence
<220>
   <223> synthetic nucleic acid
<400> 13
   gatcgcagat ccccgggtac ccgggatcct gcagtcgacg ctagggtaat acagattcga 60
   gcccaattca taaatt                                                 76
<210> 14
   <211> 68
   <212> DNA
   <213> Artificial Sequence
<220>
   <223> synthetic nucleic acid
<400> 14
   gatcgcagat ccccgggtac ccgggatcct gcagtcgacg ctacagattc gagcccaatt 60
   cataaatt 68
<210> 15
   <211> 65
   <212> DNA
   <213> Artificial Sequence
<220>
   <223> synthetic nucleic acid
<400> 15
   gatcgcagat ccccgggtac ccgggatcct gcagtcgacg cagattcgag cccaattcat 60
   aaatt 65
<210> 16
   <211> 26
   <212> DNA
   <213> Artificial Sequence
<220>
   <223> synthetic nucleic acid
<400> 16
   gatcgcagga gcccaattca taaatt 26
<210> 17
   <211> 30
   <212> DNA
   <213> Artificial Sequence
<220>
   <223> synthetic nucleic acid
<400> 17
   gatcgcagat tcgagcccaa ttcataaatt 30
<210> 18
   <211> 10
   <212> DNA
   <213> Artificial Sequence
<220>
   <223> synthetic nucleic acid
<400> 18
   gatcgcagat 10
<210> 19
   <211> 96
   <212> DNA
   <213> Nicotiana benthamiana
<400> 19
   attaatttct aatggagtag tttagtgtaa taaagttagc ttgttccaca tttttatttc 60
   ataagctatg tcatgctggg tcagattgga actcct 96
<210> 20
   <211> 93
   <212> DNA
   <213> Artificial Sequence
<220>
   <223> synthetic nucleic acid
<400> 20
   attaatttct aatggagtag tttagtgtaa taaagttagc ttgttccaca tttttattta 60
   agctatgtca tgctgggtca gattggaact cct 93
<210> 21
   <211> 92
   <212> DNA
   <213> Artificial Sequence
<220>
   <223> synthetic nucleic acid
<400> 21
   attaatttct aatggagtag tttagtgtaa taaagttagc ttgttccaca tttttatttt 60
   gctatgtcat gctgggtcag attggaactc ct 92
<210> 22
   <211> 91
   <212> DNA
   <213> Artificial Sequence
<220>
   <223> synthetic nucleic acid
<400> 22
   attaatttct aatggagtag tttagtgtaa taaagttagc ttgttccaca tttttaaaag 60
   ctatgtcatg ctgggtcaga ttggaactcc t 91
<210> 23
   <211> 90
   <212> DNA
   <213> Artificial Sequence
<220>
   <223> synthetic nucleic acid
<400> 23
   attaatttct aatggagtag tttagtgtaa taaagttagc ttgttccaca tttttatttc 60
   tatgtcatgc tgggtcagat tggaactcct                                   90
<210> 24
   <211> 88
   <212> DNA
   <213> Artificial Sequence
<220>
   <223> synthetic nucleic acid
<400> 24
   attaatttct aatggagtag tttagtgtaa taaagttagc ttgttccaca tttttatcta 60
   tgtcatgctg ggtcagattg gaactcct                                     88
<210> 25
   <211> 88
   <212> DNA
   <213> Artificial Sequence
<220>
   <223> synthetic nucleic acid
<400> 25
   attaatttct aatggagtag tttagtgtaa taaagttagc ttgttccaca tttttattta 60
   tgtcatgctg ggtcagattg gaactcct                                     88
<210> 26
   <211> 87
   <212> DNA
   <213> Artificial Sequence
<220>
   <223> synthetic nucleic acid
<400> 26
   attaatttct aatggagtag tttagtgtaa taaagttagc ttgttccaca tataagctat 60
   gtcatgctgg gtcagattgg aactcct                                      87
<210> 27
   <211> 85
   <212> DNA
   <213> Artificial Sequence
<220>
   <223> synthetic nucleic acid
<400> 27
   attaatttct aatggagtag tttagtgtaa taaagttagc ttgttccaca ttttttatgt 60
   catgctgggt cagattggaa ctcct 85
<210> 28
   <211> 82
   <212> DNA
   <213> Artificial Sequence
<220>
   <223> synthetic nucleic acid
<400> 28
   attaatttct aatggagtag tttagtgtaa taaagttagc ttgttccaca tttttattat 60
   gctgggtcag attggaactc ct 82
<210> 29
   <211> 79
   <212> DNA
   <213> Artificial Sequence
<220>
   <223> synthetic nucleic acid
<400> 29
   attaatttct aatggagtag tttagtgtaa taaagttagc ttgttccact atgtcatgct 60
   gggtcagatt ggaactcct 79
<210> 30
   <211> 77
   <212> DNA
   <213> Artificial Sequence
<220>
   <223> synthetic nucleic acid
<400> 30
   attaatttct aatggagtag tttagtgtaa taaagttagc ttgttccaca tttttattgg 60
   gtcagattgg aactcct 77
<210> 31
   <211> 40
   <212> DNA
   <213> Artificial Sequence
<220>
   <223> synthetic nucleic acid
<400> 31
   tttcataagc tatgtcatgc tgggtcagat tggaactcct 40
<210> 32
   <211> 90
   <212> DNA
   <213> Nicotiana benthamiana
<400> 32
   atgaacaaga aaaagctgaa aattcttgtt tctctcttcg ctctcaactc aatcactctc 60
   tatctctact tctcttccca ccctgatcac 90
<210> 33
   <211> 58
   <212> DNA
   <213> Nicotiana benthamiana
<400> 33
   gtttctctct tcgctctcaa ctcaatcact ctctatctct acttctcttc ccaccctg 58
<210> 34
   <211> 56
   <212> DNA
   <213> Artificial Sequence
<220>
   <223> synthetic nucleic acid
<400> 34
   gtttctctct tcgctctcaa ctcaatcact ctatctctac ttctcttccc accctg 56
<210> 35
   <211> 53
   <212> DNA
   <213> Artificial Sequence
<220>
   <223> synthetic nucleic acid
<400> 35
   gtttctctct tcgctctcaa ctcaatcata tctctacttc tcttcccacc ctg 53
<210> 36
   <211> 52
   <212> DNA
   <213> Artificial Sequence
<220>
   <223> synthetic nucleic acid
<400> 36
   gtttctctct tcgctctcaa ctcaatctat ctctacttct cttcccaccc tg 52
<210> 37
   <211> 50
   <212> DNA
   <213> Artificial Sequence
<220>
   <223> synthetic nucleic acid
<400> 37
   gtttctctct tcgctctcaa ctctctatct ctacttctct tcccaccctg 50
<210> 38
   <211> 46
   <212> DNA
   <213> Artificial Sequence
<220>
   <223> synthetic nucleic acid
<400> 38
   gtttctctct tcgctctcaa ctcaatctac ttctcttccc accctg 46
<210> 39
   <211> 43
   <212> DNA
   <213> Artificial Sequence
<220>
   <223> synthetic nucleic acid
<400> 39
   gtttctctct tcgctctcaa ctcaatcatc tcttcccacc ctg 43
<210> 40
   <211> 42
   <212> DNA
   <213> Artificial Sequence
<220>
   <223> synthetic nucleic acid
<400> 40
   gtttctctct tcgctctcaa ctcaatcact cttcccaccc tg 42
<210> 41
   <211> 35
   <212> DNA
   <213> Artificial Sequence
<220>
   <223> synthetic nucleic acid
<400> 41
   gtttctctct tcgctctact tctcttccca ccctg 35
<210> 42
   <211> 30
   <212> DNA
   <213> Artificial Sequence
<220>
   <223> synthetic nucleic acid
<400> 42
   gtttctatct ctacttctct tcccaccctg 30
<210> 43
   <211> 26
   <212> DNA
   <213> Artificial Sequence
<220>
   <223> synthetic nucleic acid
<400> 43
   gtttctctac ttctcttccc accctg 26
<210> 44
   <211> 58
   <212> DNA
   <213> Nicotiana benthamiana
<400> 44
   gtttctctct tcgctctcaa ctcaatcact ctctatctct acttctcttc ccaccctg 58
<210> 45
   <211> 54
   <212> DNA
   <213> Artificial Sequence
<220>
   <223> synthetic nucleic acid
<400> 45
   gtttctctct tcgctctcaa ctcaatctct atctctactt ctcttcccac cctg 54
<210> 46
   <211> 53
   <212> DNA
   <213> Artificial Sequence
<220>
   <223> synthetic nucleic acid
<400> 46
   gtttctctct tcgctctcaa ctcaatccta tctctacttc tcttcccacc ctg 53
<210> 47
   <211> 52
   <212> DNA
   <213> Artificial Sequence
<220>
   <223> synthetic nucleic acid
<400> 47
   gtttctctct tcgctctcaa ctcaatctat ctctacttct cttcccaccc tg 52
<210> 48
   <211> 51
   <212> DNA
   <213> Artificial Sequence
<220>
   <223> synthetic nucleic acid
<400> 48
   gtttctctct tcgctctcaa ctcaatcatc tctacttctc ttcccaccct g 51
<210> 49
   <211> 50
   <212> DNA
   <213> Artificial Sequence
<220>
   <223> synthetic nucleic acid
<400> 49
   gtttctctct tcgctctcaa ctctctatct ctacttctct tcccaccctg 50
<210> 50
   <211> 47
   <212> DNA
   <213> Artificial Sequence
<220>
   <223> synthetic nucleic acid
<400> 50
   gtttctctct tcgctctcaa ctcaatcata cttctcttcc caccctg 47
<210> 51
   <211> 46
   <212> DNA
   <213> Artificial Sequence
<220>
   <223> synthetic nucleic acid
<400> 51
   gtttctctct tcgctctcaa ctcaatctac ttctcttccc accctg 46
<210> 52
   <211> 41
   <212> DNA
   <213> Artificial Sequence
<220>
   <223> synthetic nucleic acid
<400> 52
   gtttctctct tctctctatc tctacttctc ttcccaccct g 41
<210> 53
   <211> 32
   <212> DNA
   <213> Artificial Sequence
<220>
   <223> synthetic nucleic acid
<400> 53
   gtttctctat ctctacttct cttcccaccc tg 32
<210> 54
   <211> 29
   <212> DNA
   <213> Artificial Sequence
<220>
   <223> synthetic nucleic acid
<400> 54
   gctctatctc tacttctctt cccaccctg 29
<210> 55
   <211> 9
   <212> PRT
   <213> Artificial Sequence
<220>
   <223> consensus
<400> 55
   Leu Ala Gly Leu Ile Asp Ala Asp Gly
    1               5

## Description

### TECHNICAL FIELD

This document relates to the field of plant molecular biology, and in particular provides materials and methods for creating genome-engineered plants with non-transgenic methods.

### BACKGROUND

Traditional plant breeding strategies have been developed over many years to introduce desirable traits into plant species such as increased drought tolerance and crop yield. Such strategies have the drawback that they typically require many successive rounds of crossing, and thus it can take many years to successfully alter a specific plant trait. With the advent of transgenic technologies it became possible to engineer plants with genomic alterations by introducing transgene constructs and thus circumvent the need for traditional plant breeding. However, these transgenic techniques also had several drawbacks. First, transgene insertion into the genome (such as that mediated by *Agrobacterium tumefaciens*) is largely random and can lead to multiple insertions which can cause difficulties in tracking multiple transgenes present on different chromosomes during segregation. Further, expression of the transgene can be unpredictable due to its chromosomal environment, and in many cases expression of the transgene is silenced. In addition, production of transgenic plants has proven to be a very controversial topic, with public opinion often being against the creation of such varieties - particularly where the varieties in question are crop plants that will be grown over large geographical areas and used as food for human consumption.

Methods that allow for targeted modification of the plant genome may overcome the first two of these problems, making it possible to target transgene insertions to single chromosomal sites that are conducive to gene expression, thus reducing or eliminating the possibility of multiple transgene insertions and silencing events. Targeted genome modification has been demonstrated in a number of species using engineered Zinc Finger Nucleases (ZFNs), which permit the creation of double stranded DNA break points at preselected loci and the subsequent insertion of transgenes in a targeted manner (Lloyd et al. 2005; Wright et al. 2005; Townsend et al. 2009). A variation on this technique is to simply use the ZFN to create a break point at a chosen locus and then allow repair of the DNA by NHEJ (non-homologous end joining). During this process, errors are often incorporated into the newly joined region (e.g., nucleotide deletions) and this method allows for the targeted mutagenesis of selected plant genes as well as for the insertion of transgene constructs.

### SUMMARY

The above-mentioned advances in plant genetic engineering do not necessarily allay public fears concerning the production and widespread growth of engineered plant species. A solution to this problem would therefore be a method which can precisely alter the genome of a plant in a targeted way without the use of traditional transgenic strategies. The disclosure herein provides such a solution by providing methods for targeted, non-transgenic editing of a plant genome. The methods more particularly rely on the introduction into a plant cell of sequence-specific nucleases under protein form, which are translocated to the nucleus and which act to precisely cut the DNA at a predetermined locus. Errors made during the repair of the cut permit the introduction of loss of function (or gain of function) mutations without the introduction into the genome of any exogenous genetic material.

In this way, genetically modified plant species can be produced that contain no residual exogenous genetic material.

Prior to development of the methods described herein, genetic modification of plant cells required the stable genomic integration of a transgene cassette for the expression *in vivo* of a nuclease or a DNA modifying enzyme. Such integration was typically achieved through Agrobacterium-mediated transformation of plant species. As described herein, however, consistent and reproducible genomic modification can be achieved through the introduction into a plant cell of a purified nuclease protein. This is an unexpected effect, because recombinant nucleases or purified mRNA were not considered to be sufficiently active to have a significant effect on plant chromosomal or organelle DNA. Further, this document provides new protocols for genomic modification, and also provides sequences and vectors suitable to practice the methods described herein, and to produce modified plant cells without introducing exogenous DNA.

This document describes methods for editing plant genomes using non-transgenic strategies. Sequence-specific nucleases (including ZFNs, homing endonucleases, TAL-effector nucleases, CRISPR-associated systems [Cas9]) are introduced into plant cells in the form of purified nuclease protein or as mRNA encoding the nuclease protein. In the case of CRISPR-associated systems [Cas9], the nuclease can be introduced as purified protein along with a guide RNA for target site recognition.

The functional nucleases are targeted to specific sequences, and cut the cellular DNA at predetermined loci. The DNA damage triggers the plant cell to repair the double strand break. Mistakes (e.g., point mutations or small insertions/deletions) made during DNA repair then alter DNA sequences *in vivo.*

Unlike conventional DNA transformation, the protein or RNA-based genome editing strategies described herein specifically modify target nucleic acid sequences and leave no footprint behind. Since no foreign DNA is used in these methods, this process is considered to be non-transgenic plant genome editing.

In one aspect there is provided a method for targeted genetic modification of a plant genome without inserting exogenous genetic material comprising:
(i) providing a plant cell that comprises an endogenous gene to be modified;
(ii) providing a Cas9 endonuclease protein targeted to the endogenous gene; and
(iii) transfecting the plant cell with said Cas9 endonuclease protein using biolistic or protoplast transformation, such that the Cas9 endonuclease introduces one or more double stranded DNA breaks (DSB) in the genome, to produce a plant cell or cells having a detectable targeted genomic modification without the presence of any exogenous Cas9 genetic material in the plant genome.

The sequence-specific nuclease is Cas9 endonuclease. The Cas9 endonuclease can be delivered to the plant cell in the form of a purified protein.

The sequence-specific nuclease can further contain one or more subcellular localization domains. The one or more subcellular localization domains can include an SV40 nuclear localization signal, an acidic M9 domain of hnRNPA1, a PY-NLS motif signal, a mitochondrial targeting signal, or a chloroplast targeting signal. The sequence-specific nuclease can further contain one or more cell penetrating peptide domains (CPPs). The one or more CPPs can include a transactivating transcriptional activator (Tat) peptide or a Pep-1 CPP domain.

The sequence-specific nuclease can be co-transfected with one or more plasmids encoding one or more exonucleases. The one or more exonucleases can include a member of the TREX exonuclease family (e.g., TREX2).

The endogenous gene to be modified can be an acetolactate synthase gene (e.g., ALS1 or ALS2), or a vacuolar invertase gene (e.g., the potato (*Solanum tuberosum*) vacuolar invertase gene (VInv).

The plant cell can be from a field crop species of alfalfa, barley, bean, corn, cotton, flax, pea, rape, rice, rye, safflower, sorghum, soybean, sunflower, tobacco, wheat. The plant cell can be from the genus *Nicotiana,* or from the species *Arabidopsis thaliana.*

Transfection can be effected through delivery of the sequence-specific nuclease into isolated plant protoplasts. For example, transfection can be effected delivery of the sequence-specific nuclease into isolated plant protoplasts using polyethylene glycol (PEG) mediated transfection, electroporation, biolistic mediated transfection, sonication mediated transfection, or liposome mediated transfection.

We describe a transformed plant cell obtainable according to the methods provided herein, as well as a transformed plant containing the plant cell.

In another aspect there is provided a kit for the targeted genetic modification of a plant genome without inserting exogenous genetic material, said kit comprising:
(i) one or more Cas9 proteins;
(ii) one or more plant protoplasts or whole cultured plant cells;
   and optionally
(iii) one or more DNA plasmid vectors encoding one or more TREX family exonucleases.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### BRIEF DESCRIPTION OF THE DRAWINGS

**Figure 1** is a diagram depicting a structural organization of the sequence-specific nucleases used for genome engineering. The nucleases contain domains that function to enable cell penetration, sub-cellular protein localization, DNA sequence recognition, and DNA cleavage.
**Figure 2** is a picture showing SDS-PAGE of transcription activator-like effector endonucleases (TALEN™) produced in *E. coli.* ALS2T1L and ALS2T1R are TALEN™s that target a site in the *Nicotiana benthamiana* ALS2 gene. VInv7 is a compact TALEN™ (cT) that targets the potato VInv gene.
**Figure 3** is a picture of an agarose gel showing *in vitro* activity of purified TALEN™s targeting the *N. benthamiana* ALS2 gene. A PCR product was generated that contains the target site for the ALS2T1 TALEN™s. The PCR product was incubated without (-) or with (+) the purified ALS2T1L and ALS2T1R proteins. Only in the presence of the two proteins was the PCR product cleaved. As a negative control, the purified ALS2T1L and ALS2T1R proteins were incubated with a PCR product from the ALS1 gene; no cleavage was observed.
**Figure 4** is a table showing the activity of I-Scel on episomal targets when delivered to plant cells as a protein.
**Figure 5** is a table showing activity of I-SceI activity on a chromosomal site when delivered to plant cells as a protein alone or in combination with TreX. The number in the total number of 454 sequencing reads used for this analysis is indicated in parentheses in column 2.
**Figure 6** is a sequence alignment showing examples of I-SceI-induced mutations in a transgenic *N. tabacum* line that contains an integrated I-SceI recognition site. The top line (SEQ ID NO:8) indicates the DNA sequence of the recognition site for I-SceI (underlined). The other sequences (SEQ ID NOS:9 to 18) show representative mutations that were induced by imprecise non-homologous end-joining (NHEJ).
**Figure 7** is a graph summarizing TALEN™ ALS2T1 mutagenesis activity after transformation into plant cells in different forms (DNA or protein) or treatment combinations.
**Figure 8** is a sequence alignment showing examples of TALEN™ ALS2T1 induced mutations in the *N. benthamiana* ALS2 gene. The top line (SEQ ID NO:19) shows the DNA sequence of the recognition site for ALS2T1 (underlined). The other sequences (SEQ ID NOS:20 to 31) show representative mutations that were induced by imprecise non-homologous end-joining (NHEJ).
**Figure 9** is a diagram showing the structural organization of a sequence-specific nuclease as used for *in vitro* mRNA production. The nuclease construct contains a T7 promoter, a nuclease ORF, and a 121-bp polyA tail.
**Figure 10** is a graph plotting the cleavage activity of I-Crel mRNA delivered to plant protoplasts in a YFP-based SSA assay. A SSA target plasmid together with p35S-I-CreI or I-CreI mRNA was co-delivered to tobacco protoplasts via PEG-mediated transformation. Twenty four hours after transformation, the protoplasts were subjected to flow cytometry to quantify the number of YFP-positive cells.
**Figure 11** is the target sequence (SEQ ID NO:32) for the XylT_T04 TALEN™ in *N. benthamiana.*
**Figure 12** is a table recapitulating the 454 pyro-sequencing data for delivery of Xyl_T04 TALEN™ mRNA to tobacco protoplasts. The numbers in parenthesis in column 3 are the total number of sequencing reads obtained *: NHEJ mutagenesis frequency was obtained by normalizing the percentage of 454 reads with NHEJ mutations to the protoplast transformation efficiency. The total number of 454 sequencing reads used for this analysis is indicated in parentheses. **: Negative controls were obtained from protoplasts transformed only by the YFP-coding plasmid.
**Figure 13** is a sequence alignment showing examples of mutations induced by XylT_T04 mRNA in *N. benthamiana* at the XylT1 (SEQ ID NOS:33 to 43) and XylT2 (SEQ ID NOS:44 to 54) genes.

### DETAILED DESCRIPTION

This document provides new strategies for editing plant genomes to generate non-transgenic plant material. The methods provided herein are carried out using a nuclease designed to recognize specific sequences in any site of the plant genome.

In one aspect, this document relates to a method for targeted genetic modification of a plant genome without inserting exogenous genetic material comprising one or several of the following steps:
i) providing a plant cell which comprises an endogenous gene to be modified;
ii) obtaining a sequence-specific nuclease comprising a sequence recognition domain and a nuclease domain;
iii) transformation of the plant cell with said sequence-specific nuclease, and
iv) induction of one or more double stranded DNA breaks in the genome;

This method aims at producing a plant cell or cells having a detectable targeted genomic modification, preferably without the presence of any exogenous genetic material in the plant genome.

Induction of double stranded breaks in the genome generally leads to repair of the breaks by non homologous end joining (NHEJ), which favours deletion, correction or insertion of genetic sequences into the genome of the plant cell obtained by the method.

After the coding sequence for the nuclease has been synthesized and cloned into an expression vector, nuclease protein or mRNA is produced and purified. To improve efficacy, cell penetrating peptides (CPP) can be added to improve cell membrane permeability for small molecules (drugs), proteins and nucleic acids (Mäe and Langel, 2006; US 2012/0135021, US 2011/0177557, US 7,262,267). Sub-cellular localization peptides can also be added to direct protein traffic in cells, particularly to the nucleus (Gaj et al. 2012; US 20050042603).

In some embodiments, a protein with exonuclease activity, such as, for example, Trex (WO 2012/058458) and/or Tdt (Terminal deoxynucleotidyl transferase) (WO 2012/13717), is co-delivered to the plant cell for increasing sequence-specific nuclease induced mutagenesis efficiency. Trex2 (SEQ ID NO:6) has shown to be particularly effective by increasing mutagenesis as described herein. Using Trex2 expressed as a single polypeptide chain (SEQ ID NO:7) was even more effective.

Purified nucleases are delivered to plant cells by a variety of means. For example, biolistic particle delivery systems may be used to transform plant tissue. Standard PEG and/or electroporation methods can be used for protoplast transformation. After transformation, plant tissue/cells are cultured to enable cell division, differentiation and regeneration. DNA from individual events can be isolated and screened for mutation.

The document describes that the sequence-specific nuclease is a TAL-effector nuclease (Beurdeley et al., 2013). It is also envisaged that any type of sequence-specific nuclease may be used to perform the methods provided herein as long as it has similar capabilities to TAL-effector nucleases. Therefore, it must be capable of inducing a double stranded DNA break at one or more targeted genetic loci, resulting in one or more targeted mutations at that locus or loci where mutation occurs through erroneous repair of the break by NHEJ or other mechanism (Certo et al., 2012). Such sequence-specific nucleases include, but are not limited to, ZFNs, homing endonucleases such as I-SceI and I-CreI, restriction endonucleases and other homing endonucleases or TALEN™s. In a specific embodiment, the endonuclease to be used comprises a CRISPR-associated Cas protein, such as Cas9 (Gasiunas et al., 2012).

The document describes that the sequence-specific nuclease to be delivered may be either in the form of purified nuclease protein, or in the form of mRNA molecules which can are translated into protein after transfection. Nuclease proteins may be prepared by a number of means known to one skilled in the art, using available protein expression vectors such as, but not limited to, pQE or pET. Suitable vectors permit the expression of nuclease protein in a variety of cell types (*E. coli,* insect, mammalian) and subsequent purification. Synthesis of nucleases in mRNA format may also be carried out by various means known to one skilled in the art such as through the use of the T7 vector (pSF-T7) which allows the production of capped RNA for transfection into cells.

The mRNA may be modified with optimal 5' untranslated regions (UTR) and 3' untranslated regions. UTRs have been shown to play a pivotal role in post-translational regulation of gene expression via modulation of localization, stability and translation efficiency (Bashirullah, 2001). As noted above, mRNA delivery is desirable due to its non-transgenic nature; however, mRNA is a very fragile molecule, which is susceptible to degradation during the plant transformation process. Utilization of UTRs in plant mRNA transformations allow for increased stability and localization of mRNA molecules, granting increased transformation efficiency for non-transgenic genome modification.

In some embodiments, the engineered nuclease includes one or more subcellular localization domains, to allow the efficient trafficking of the nuclease protein within the cell and in particular to the nucleus (Gaj. et al., 2012; US 2005/0042603). Such a localization signal may include, but is not limited to, the SV40 nuclear localization signal (Hicks et al., 1993). Other, non-classical types of nuclear localization signal may also be adapted for use with the methods provided herein, such as the acidic M9 domain of hnRNP A1 or the PY-NLS motif signal (Dormann et al., 2012). Localization signals also may be incorporated to permit trafficking of the nuclease to other subcellular compartments such as the mitochondria or chloroplasts. Guidance on the particular mitochondrial and chloroplastic signals to use may be found in numerous publications (*see,* Bhushan S. et al., 2006) and techniques for modifying proteins such as nucleases to include these signals are known to those skilled in the art.

In some embodiments, the nuclease includes a cell-penetrating peptide region (CPP) to allow easier delivery of proteins through cell membranes (Mäe and Langel, 2006; US 2012/0135021, US 2011/0177557, US 7,262,267). Such CPP regions include, but are not limited to, the transactivating transcriptional activator (Tat) cell penetration peptide (Lakshmanan et al., 2013, Frankel et al., 1988). It is envisaged that other CPP's also may be used, including the Pep-1 CPP region, which is particularly suitable for assisting in delivery of proteins to plant cells (*see,* Chugh et al., 2009).

In some embodiments, one or more mutations are generated in the coding sequence of one of the acetolactate synthase (ALS) genes ALS1 or ALS2, or one or more mutations are generated in the vacuolar invertase (VInv) gene. In a further aspect of these embodiments, the mutation may be any transition or transversion which produces a non-functional or functionally-reduced coding sequence at a predetermined locus. It is generally envisaged that one or more mutations may be generated at any specific genomic locus using the methods described herein.

In some embodiments, the plant species used in the methods provided herein is *N. benthamiana,* although in a further aspect the plant species may be any monocot or dicot plant, such as (without limitation) *Arabidopsis thaliana;* field crops (e.g., alfalfa, barley, bean, corn, cotton, flax, pea, rape, rice, rye, safflower, sorghum, soybean, sunflower, tobacco, and wheat); vegetable crops (e.g., asparagus, beet, broccoli, cabbage, carrot, cauliflower, celery, cucumber, eggplant, lettuce, onion, pepper, potato, pumpkin, radish, spinach, squash, taro, tomato, and zucchini); fruit and nut crops (e.g., almond, apple, apricot, banana, blackberry, blueberry, cacao, cherry, coconut, cranberry, date, fajoa, filbert, grape, grapefruit, guava, kiwi, lemon, lime, mango, melon, nectarine, orange, papaya, passion fruit, peach, peanut, pear, pineapple, pistachio, plum, raspberry, strawberry, tangerine, walnut, and watermelon); and ornamentals (e.g., alder, ash, aspen, azalea, birch, boxwood, camellia, carnation, chrysanthemum, elm, fir, ivy, jasmine, juniper, oak, palm, poplar, pine, redwood, rhododendron, rose, and rubber).

In some embodiments, the protein or mRNA encoding the nuclease construct is delivered to the plant cells via PEG-mediated transformation of isolated protoplasts. PEG typically is used in the range from half to an equal volume of the mRNA or protein suspension to be transfected, PEG40% being mostly used in this purpose.

In some cases, the nuclease may be delivered via through biolistic transformation methods or through any other suitable transfection method known in the art (Yoo et al, 2007). In the case of biolistic transformation, the nuclease can be introduced into plant tissues with a biolistic device that accelerates the microprojectiles to speeds of 300 to 600 m/s which is sufficient to penetrate plant cell walls and membranes (*see,* Klein et al., 1992). Another method for introducing protein or RNA to plants is via the sonication of target cells.

Alternatively, liposome or spheroplast fusion may be used to introduce exogenous material into plants (*see,* e.g., Christou et al., 1987). Electroporation of protoplasts and whole cells and tissues has also been described (Laursen et al., 1994).

Depending on the method used for transfection and its efficiency, the inventors determined that the optimal protein concentration for performing the methods described herein, especially with TALEN™s, was between 0.01 to 0.1 µg/µl. When using PEG, the volume of the protein suspension was generally between 2 to 20 µl. RNA concentration was found optimal in the range of 1 to 5 µg/µl, it being considered that it can be sometimes advantageous to add non coding RNA, such as tRNA carrier, up to 10 µg/µl, in order to increase RNA bulk. This later adjunction of RNA improves transfection and has a protective effect on the RNA encoding the nuclease with respect to degradative enzymes encountered in the plant cell.

A plant can be obtained by regenerating the plant cell produced by any of the method described herein. When the function of the endogenous gene is suppressed in the plant cell into which the non-silent mutation is introduced at the target DNA site, the phenotype of the plant regenerated from such a plant cell may be changed in association with the suppression of the function of the endogenous gene. Accordingly, the methods described herein make it possible to efficiently perform breeding of a plant. The regeneration of a plant from the plant cell can be carried out by a method known to those skilled in the art which depends on the kind of the plant cell. Examples thereof include the method described in Christou et al. (1997) for transformation of rice species.

In some embodiments, the nuclease can be co-delivered with a plasmid encoding one or more exonuclease proteins to increase sequence-specific nuclease induced mutagenesis efficiency. Such exonucleases include, but are not limited to, members of the Trex family of exonucleases (Therapeutic red cell exchange exonucleases) such as TREX2 (Shevelev et al. 2002). The inventors have surprisingly found that co-delivery of an exonuclease such as TREX with purified I-SceI protein increases the frequency of NHEJ events observed as compared with delivery of the I-SceI protein alone. It is to be noted that other suitable exonucleases may also be used in the methods provided herein.

As used herein the term "identity" refers to sequence identity between two nucleic acid molecules or polypeptides. Identity is determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Alignment algorithms and programs are used to calculate the identity between two sequences. FASTA and BLAST are available as a part of the GCG sequence analysis package (University of Wisconsin, Madison, WI), and are used with default setting. BLASTP may also be used to identify an amino acid sequence having at least 80%, 85%, 87.5%, 90%, 92.5%, 95%, 97.5%, 98%, or 99% sequence similarity to a reference amino acid sequence using a similarity matrix such as BLOSUM45, BLOSUM62 or BLOSUM80. Unless otherwise indicated, a similarity score is based on use of BLOSUM62. When BLASTP is used, the percent similarity is based on the BLASTP positives score and the percent sequence identity is based on the BLASTP identities score. BLASTP "Identities" shows the number and fraction of total residues in the high scoring sequence pairs which are identical; and BLASTP "Positives" shows the number and fraction of residues for which the alignment scores have positive values and which are similar to each other. Amino acid sequences having these degrees of identity or similarity or any intermediate degree of identity of similarity to the amino acid sequences disclosed herein are contemplated and encompassed by this disclosure. The same applies with respect to polynucleotide sequences using BLASTN.

As used herein the term "homologous" is intended to mean a sequence with enough identity to another one to lead to a homologous recombination between sequences, more particularly having at least 95% identity (e.g., at least 97% identity, or at least 99% identity).

As used herein the term "endonuclease" refers to an enzyme capable of causing a double-stranded break in a DNA molecule at highly specific locations.

As defined herein the term "exonuclease" refers to an enzyme that works by cleaving nucleotides one at a time from the end (exo) of a polynucleotide chain causing a hydrolyzing reaction that breaks phosphodiester bonds at either the 3' or the 5' to occur.

As used herein the term "sequence-specific nuclease" refers to any nuclease enzyme which is able to induce a double-strand DNA break at a desired and predetermined genomic locus

As used herein the term "meganuclease" refers to natural or engineered rare-cutting endonuclease, typically having a polynucleotide recognition site of about 12-40 bp in length, more preferably of 14-40 bp. Typical meganucleases cause cleavage inside their recognition site, leaving 4 nt staggered cut with 3'OH overhangs. The meganuclease are preferably homing endonuclease, more particularly belonging to the dodecapeptide family (LAGLIDADG; SEQ ID NO:55) (WO 2004/067736), TAL-effector like endonuclease , zinc-finger-nuclease, or any nuclease fused to modular base-per-base binding domains (MBBBD) - i.e., endonucleases able to bind a predetermined nucleic acid target sequence and to induce cleavage in sequence adjacent thereto . These meganucleases are useful for inducing double-stranded breaks in specific DNA sequences and thereby promote site-specific homologous recombination and targeted manipulation of genomic sequences.

As used herein the term "vector" refers to a nucleic acid molecule capable of transporting another nucleic acid to which it has been linked into a cell, or a cell compartment.

As used herein the term "zinc finger nuclease" refers to artificial restriction enzymes generated by fusing a zinc finger DNA-binding domain to a DNA-cleavage domain. Briefly, ZFNs are synthetic proteins comprising an engineered zinc finger DNA-binding domain fused to the cleavage domain of the FokI restriction endonuclease. ZFNs may be used to induce double-stranded breaks in specific DNA sequences and thereby promote site-specific homologous recombination and targeted manipulation of genomic sequences.

As used herein the term "TAL-effector endonuclease" refers to artificial restriction enzymes generated by fusing a DNA recognition domain deriving from TALE proteins of *Xanthomonas* to a catalytic domain of a nuclease, as described by Voytas and Bogdanove in WO 2011/072246. TAL-effector endonucleases are named TALEN™ by the applicant (Cellectis, 8 rue de la Croix Jarry, 75013 PARIS).

The invention will be further described in the following reference examples, which do not limit the scope of the invention described in the claims.

### EXAMPLES

### Reference Example 1: Designing and constructing sequence-specific nucleases for protein expression.

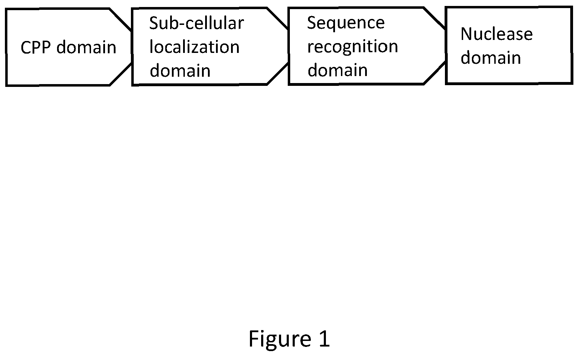

A sequence-specific nuclease typically includes the following components (Figure 1):
1. A DNA binding domain that recognizes a specific DNA sequence in a plant genome.
2. A nuclease domain that creates a DNA double-strand break at the recognition site in the plant genome. Imprecise repair of the break through non-homologous end-joining introduces mutations at the break site.
3. A sub-cellular localization signal that directs the nuclease to the nucleus, mitochondria or chloroplast.
4. A cell penetration motif that helps the sequence-specific nuclease penetrate cell membranes during transformation.

Sequences encoding the custom nuclease may be synthesized and cloned into a protein expression vector, such as pQE or pET. Functional protein can therefore be expressed in E. coli and purified using standard protocols or commercial kits. Alternatively, other protein expression systems, including yeast, insect or mammalian cells, can be used to produce proteins that are difficult to express and purify in *E. coli.*

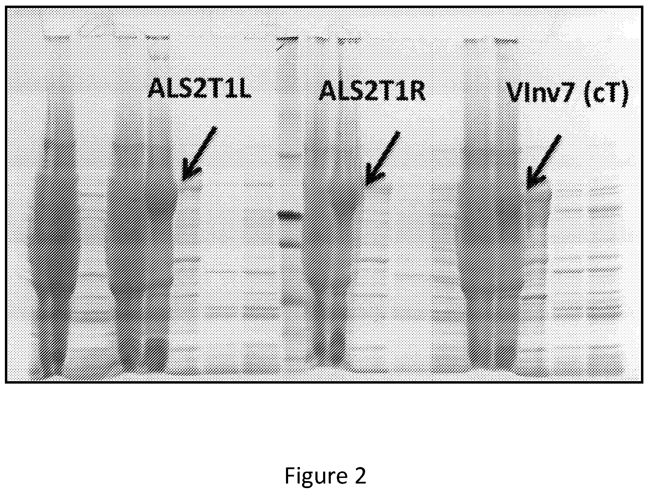

Here, pQE-80L-Kan was used as the protein expression vector. An SV40 nuclear localization signal was added as well as the Tat cell penetration peptide (Frankel and Pabo, 1988, Schwarze et al., 1999). Sequence specific nucleases included a TALEN™ pair targeting a site in the *N. benthamiana* ALS2 gene. In addition, a compact TALEN™ was also used that targets a site in the VInv7 gene in S. *tuberosum. E. coli* strain BL21 was used for protein expression (Beurdeley et al., 2013). A Qiagen Ni-NTA Spin kit was used for protein purification. A high yield of recombinant protein was obtained for all three TALEN™ in *E. coli* (Figure 2). The plasmids for producing the recombinant TALEN™ were provided by Cellectis Bioresearch (8, rue de la Croix Jarry, 75013 PARIS).

### Reference Example 2: In vitro sequence-specific nuclease activity of purified TALEN™.

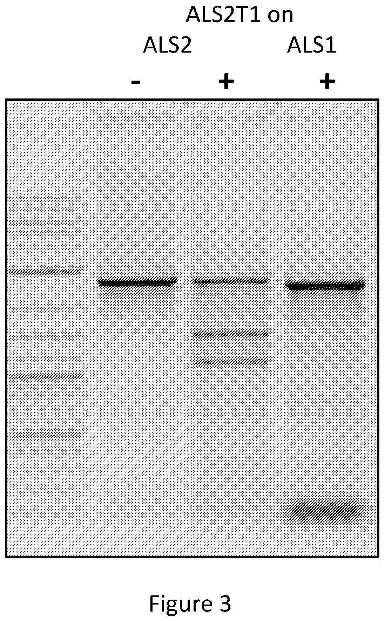

To test the enzyme activity of purified TALEN™, equal amounts of ALS2T1L (SEQ ID NO:2) and ALS2T1R (SEQ ID NO:3) proteins were mixed and incubated with a PCR fragment derived from the *N. benthamiana* ALS2 gene (the PCR product has the TALEN recognition site). The reaction was carried out at 25°C and had the following buffer system: 100mM NaCl, 50mM Tris-HCl, 10mM MgCl₂, and ImM dithiothreitol, pH 7.9. A PCR fragment derived from the *N. benthamiana* ALS1 gene (lacking the TALEN recognition site) was used as a negative control. The two TALENs clearly cleaved the ALS2 gene fragment *in vitro;* no activity was observed with the ALS1 fragment (Figure 3). The data indicate that the purified TALEN™ have sequence-specific nuclease activity.

### Reference Example 3: Delivery of sequence-specific nucleases to plant cells as proteins.

The enzyme I-SceI was purchased from New England Biolabs and dialyzed to remove the buffer supplied by the manufacturer. Briefly, 20µl (100U) of I-Scel was placed on a Millipore 0.025µm VSWP filter (CAT# VSWP02500). The filter was floated on a MMG buffer (0.4 M mannitol, 4 mM MES, 15 mM MgCl₂, pH 5.8) at 4°C for 1 hour. After the enzyme solution was completely equilibrated by MMG buffer, it was transferred to a new tube and kept on ice until further use. The protein was delivered to plant cells by PEG-mediated protoplast transformation. Methods for tobacco protoplast preparation were as previously described (Zhang et al. 2013). Briefly, seeds from a transgenic tobacco line with an integrated I-SceI recognition site were planted in moistened vermiculite and grown under low light conditions for 3-5 weeks (Pacher et al., 2007). Young, fully expanded leaves were collected and surface sterilized, and protoplasts were isolated.

Purified I-SceI protein was introduced into N. tabacum protoplasts by PEG-mediated transformation as described elsewhere (Yoo et al., Nature Protocols 2:1565-1572, 2007). Briefly, 20-200U of I-Scel protein was mixed with 200,000 protoplasts at room temperature in 200µl of 0.4 M mannitol, 4 mM MES, 15 mM MgCl₂, pH 5.8. Other treatments included transforming protoplasts with DNA that encodes I-SceI (SEQ ID NO:1), DNA that encodes the Trex2 protein, or both. Trex2 is an endonuclease that increases frequencies of imprecise DNA repair through NHEJ (Certo et al. 2012). In addition, in one sample, I-SceI protein was co-delivered with Trex2-encoding DNA (SEQ ID NO:6). After transformation, an equal volume of 40% PEG-4000, 0.2 M mannitol, 100 mM CaCl₂, pH5.8 was added to protoplasts and immediately mixed well. The mixture was incubated in the dark for 30 minutes before washing once with 0.45 M mannitol, 10 mM CaCl₂. The protoplasts were then washed with K3G1 medium twice before moving the cells to 1 ml of K3G1 in a petri dish for long-term culture.

### Reference Example 4: Activity of I-SceI on episomal target sites in N. tabacum.

To assess the protein activity of the I-SceI targeting episomal sites in plant cell, a SSA construct was co-delivered with I-SceI protein. For this assay, a target plasmid was constructed with the I-Sce recognition site cloned in a non-functional YFP reporter gene. The target site was flanked by a direct repeat of YFP coding sequence such that if the reporter gene was cleaved by the I-Sce, recombination would occur between the direct repeats and function would be restored to the YFP gene. Expression of YFP, therefore, served as a measure of I-Scel cleavage activity.

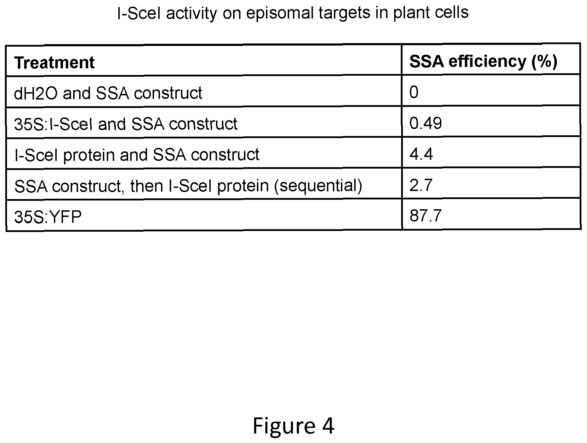

The activity of the I-SceI protein and its control treatments against the episomal target sequence is summarized in Figure 4. The delivery of I-Scel as DNA yielded a 0.49% YFP expression; while I-SceI protein yielded 4.4% expression of YFP. When the SSA construct and the I-SceI protein were delivered sequentially, the YFP SSA efficiency was 2.4%. The transformation efficiency was indicated by YFP expression of 35S:YFP DNA delivery control.

### Reference Example 5: Activity of I-SceI at their endogenous target sites in N. tabacum.

A transgenic tobacco line contains a single I-SceI recognition site in the genome as described previously (Pacher et al., 2007). Transformed protoplasts isolated from this transgenic line were harvested 24-48 hours after treatment, and genomic DNA was prepared. Using this genomic DNA as a template, a 301 bp fragment encompassing the I-SceI recognition site was amplified by PCR. The PCR product was then subjected to 454 pyro-sequencing. Sequencing reads with insertion/deletion (indel) mutations in the recognition site were considered as having been derived from imprecise repair of a cleaved I-SceI recognition site by NHEJ. Mutagenesis frequency was calculated as the number of sequencing reads with NHEJ mutations out of the total sequencing reads.

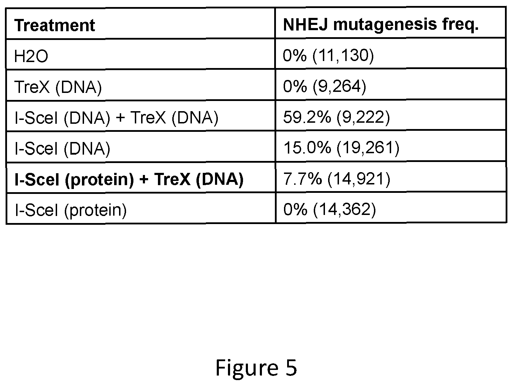

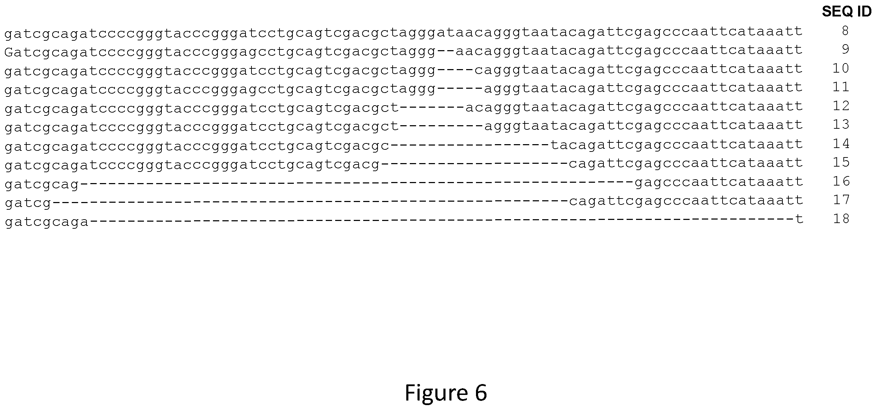

The activity of the I-SceI protein and its control treatments against the target sequence is summarized in Figure 5. The delivery of I-Scel as DNA (SEQ ID NO:1) yielded a 15% mutagenesis frequency. When combined with DNA encoding the exonuclease Trex2, the mutagenesis frequency increased to 59.2%. When I-SceI was delivered as protein, the mutagenesis activity was undetectable; however, when I-SceI protein was co-delivered with Trex2-encoding DNA (SEQ ID NO:6), a 7.7% mutagenesis frequency was observed. Examples of I-Scel protein induced mutations are shown in Figure 6. Collectively, the data demonstrate that I-SceI protein creates targeted chromosome breaks when delivered to plant cells as protein. Further, the imprecise repair of these breaks leads to the introduction of targeted mutations.

### Reference Example 6: Delivery of TALEN proteins to plant cells.

Purified TALEN™ protein was introduced into *N. benthamiana* protoplasts by PEG-mediated transformation as described in Example 4. Briefly, 2-20µl of ALS2T1 protein was mixed with 200,000 protoplasts at room temperature in 200µl of 0.4 M mannitol, 4 mM MES, 15 mM MgCl₂, pH 5.8. Other treatments included transforming protoplasts with combination of Trex2 protein, DNA that encodes ALS2T1, DNA that encodes the Trex2 protein, or a DNA construct for YFP expression. Trex2 is an endonuclease that increases frequencies of imprecise DNA repair through NHEJ. After transformation, an equal volume of 40% PEG-4000, 0.2 M mannitol, 100 mM CaCl₂, pH5.8 was added to protoplasts and immediately mixed well. The mixture was incubated in the dark for 30 minutes before washing once with 0.45 M mannitol, 10 mM CaCl₂. The protoplasts were then washed with K3G1 medium twice before moving the cells to 1 ml of K3G1 in a petri dish for long-term culture.

### Reference Example 7: Activity of TALEN™ ALS2T1 at their endogenous target sites in N. benthamiana.

Transformed protoplasts were harvested 48 hours after treatment, and genomic DNA was prepared. Using this genomic DNA as a template, a 253 bp fragment encompassing the ALS2T1 recognition site was amplified by PCR. The PCR product was then subjected to 454 pyro-sequencing. Sequencing reads with insertion/deletion (indel) mutations in the recognition site were considered as having been derived from imprecise repair of a cleaved I-SceI recognition site by NHEJ. Mutagenesis frequency was calculated as the number of sequencing reads with NHEJ mutations out of the total sequencing reads.

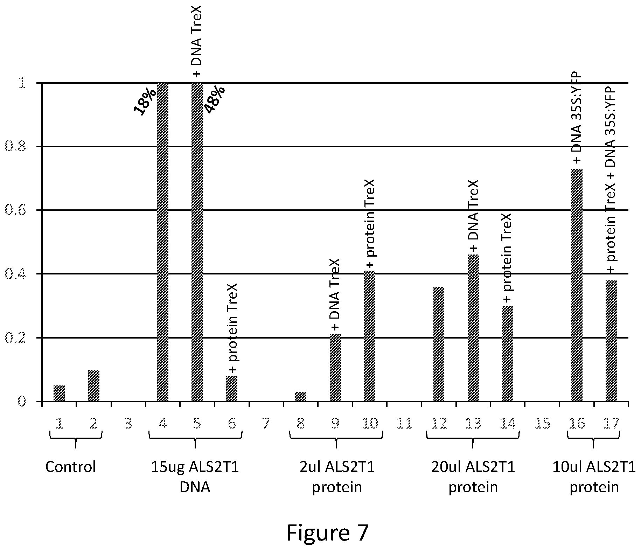

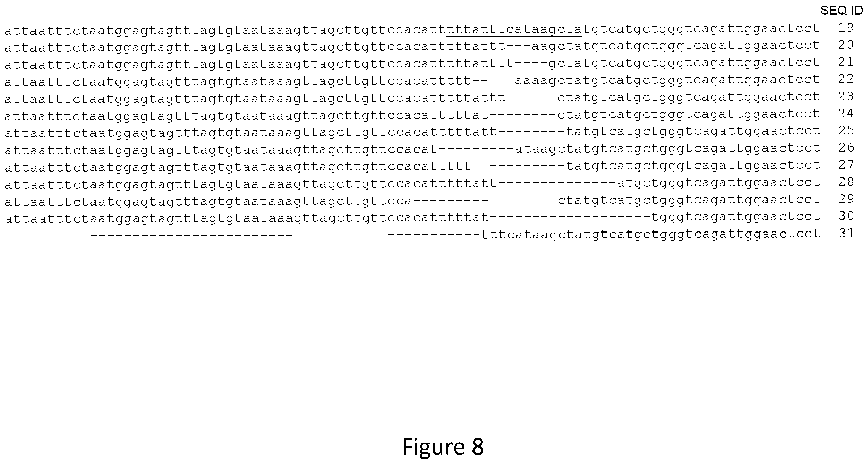

The activity of the ALS2T1 protein and its control treatments against the target sequence is summarized in Figure 7. The delivery of ALS2T1 as DNA (SEQ ID NOS:2 and 3) yielded an 18.4% mutagenesis frequency. When combined with DNA encoding the exonuclease Trex2 (SEQ ID NO:6), the mutagenesis frequency increased to 48%. When ALS2T1 was delivered as protein, the mutagenesis activity was 0.033% to 0.33%; however, when ALS2T1 protein was co-delivered with 35S:YFP DNA, a 0.72% mutagenesis frequency was observed. Examples of ALS2T1 protein-induced mutations are shown in Figure 8. Collectively, the data demonstrate that ALS2T1 protein creates targeted chromosome breaks when delivered to plant cells as protein. Further, the imprecise repair of these breaks leads to the introduction of targeted mutations.

### Reference Example 8: Preparation of mRNA encoding sequence-specific nucleases.

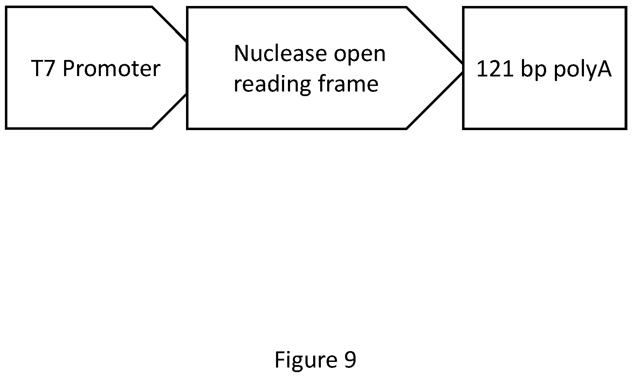

Sequence-specific nucleases, including meganucleases, zinc finger nucleases (ZFNs), or transcription activator-like effector nucleases (TALEN™), are cloned into a T7 expression vector (Figure 9). Several different 5' and 3' UTR pairs were chosen based on data from a genome wide study of transcript decay rates in *A*. *thaliana* (Narsai, 2007). The sequences selected were based on the half-lives of various transcripts as well as the functional categories. These UTR pairs were synthesized to allow convenient cloning into the T7-driven plasmid vector. The resulting nuclease constructs are linearized by SapI digestion; a SapI site is located right after the polyA sequences. The linearized plasmid serves as the DNA template for *in vitro* mRNA production using the T7 Ultra kit (Life Technologies Corporation). Alternatively, mRNA encoding the nuclease can be prepared by a commercial provider. Synthesized mRNAs are dissolved in nuclease-free distilled water and stored at - 80°C.

### Reference Example 9: Activity on episomal targets of sequence-specific nucleases delivered as mRNA.

A single-strand annealing (SSA) assay was used to measure activity of nuclease mRNAs that had been transformed into tobacco protoplasts (Zhang et al. 2013). As described in Example 4, the SSA assay uses a non-functional YFP reporter that is cleaved by the nuclease. Upon cleavage, recombination between repeated sequences in the reporter reconstitutes a functional YFP gene. YFP fluorescence can then be quantified by flow cytometry.

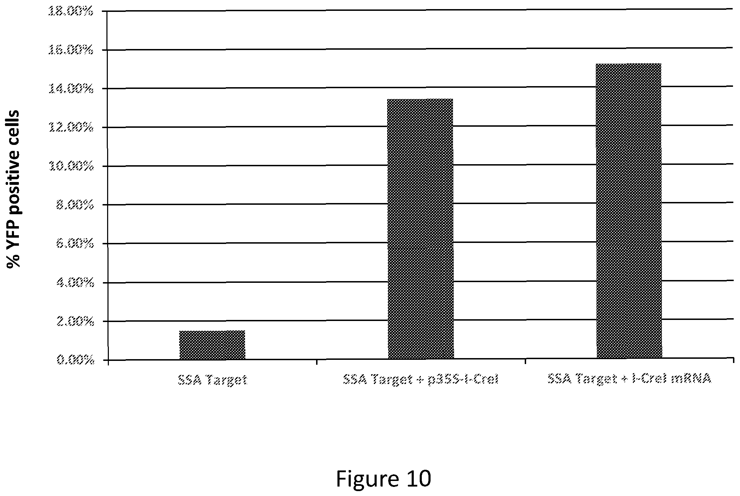

To determine whether mRNA could be delivered to plant cells and mediate targeted DNA modification, I-CreI mRNAs together with a SSA target plasmid were introduced into tobacco protoplasts by PEG-mediated transformation (Golds et al. 1993, Yoo et al. 2007, Zhang et al. 2013). The SSA reporter has an I-CreI site between the repeated sequences in YFP. Methods for tobacco protoplast preparation and transformation were as previously described (Zhang et al. 2013). The SSA target plasmid alone served as a negative control. As a positive control, cells were transformed with a DNA construct expressing I-CreI (p35S-I-CreI) as well as the I-CreI SSA reporter. Twenty-four hours after transformation, YFP fluorescence was measured by flow cytometry (FIG 10). Similar levels of targeted cleavage of the SSA reporter were observed both with p35S-I-CreI DNA and I-CreI mRNA. The data demonstrate that functional nucleases can be successful delivered to protoplasts in the form of mRNA.

### Reference Example 10: Cleavage activity on chromosomal targets of sequence-specific nucleases delivered as mRNA.

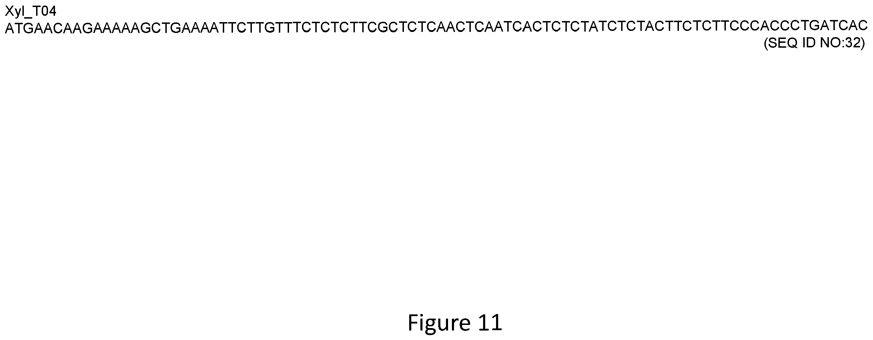

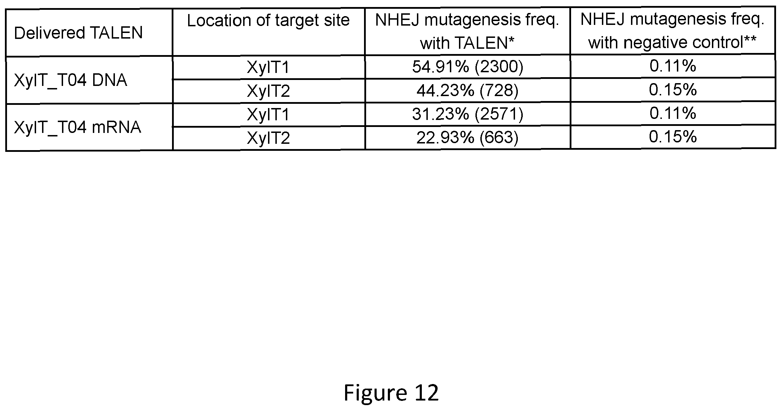

A TALEN pair (XylT TALEN™) was designed to cleave the endogenous β1,2-xylosyltransferase genes of *N. benthamiana* (Strasser et al. 2008) (Figure 11). These genes are designated XylT1 and XylT2, and the TALEN™ recognizes the same sequence found in both genes. Each XylT TALEN™ was subcloned into a T7-driven expression plasmid (Figure 12). The resulting TALEN™ expression plasmids were linearized by SapI digestion and served as DNA templates for *in vitro* mRNA production as described in Example 8.

TALEN™-encoding plasmid DNA or mRNA were next introduced into N. *benthamiana* protoplasts by PEG-mediated transformation (Golds et al., 1993, Yoo et al. 2007, Zhang et al., 2013). Protoplasts were isolated from well-expanded leaves of one month old *N. benthamiana.* The protoplast density was adjusted to the cell density of 5 x 10⁵/ml to 1x10⁶/ml, and 200 µl of protoplasts were used for each transformation. For mRNA delivery, an RNA cocktail was prepared by mixing 15 µl of L-TALEN mRNA (2µg/µl), 15 µl of R-TALEN mRNA (2µg/µl), and 10 µl of yeast tRNA carrier (10µg/µl). To minimize the potential degradation by RNAse, the RNA cocktail was quickly added to 200 µl of protoplasts, and gently mixed only for a few seconds by finger tapping. Almost immediately, 210 µl of 40% PEG was added, and mixed well by finger tapping for 1 min. The transformation reaction was incubated for 30 min at room temperature. The transformation was stopped by the addition of 900 µl of wash buffer. After a couple of washes, transformed protoplasts were cultured in K3/G1 medium at the cell density of 5x10⁵/ml. The TALEN-encoding plasmid DNA was also transformed as a positive control.

Three days after treatment, transformed protoplasts were harvested, and genomic DNA was prepared. Using the genomic DNA prepared from the protoplasts as a template, an approximately 300-bp fragment encompassing the TALEN™ recognition site was amplified by PCR. The PCR product was then subjected to 454 pyro-sequencing. Sequencing reads with insertion/deletion (indel) mutations in the spacer region were considered as having been derived from imprecise repair of a cleaved TALEN™ recognition site by non-homologous end-joining (NHEJ). Mutagenesis frequency was calculated as the number of sequencing reads with NHEJ mutations out of the total sequencing reads.

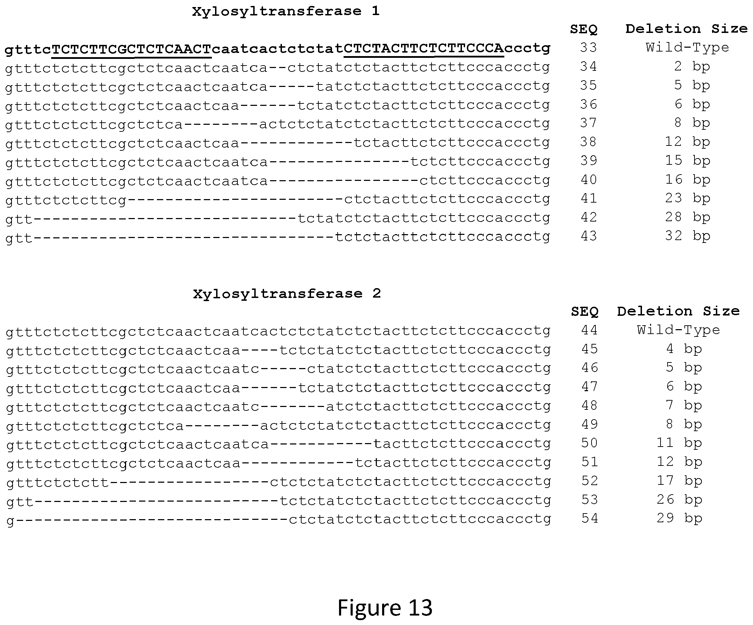

Xyl_T04 TALEN™ DNA and mRNA were tested against their targets, namely the XylT1 and XylT2 genes in *N. benthamiana.* As indicated above, the TALEN™ recognition sites are present in both XylT1 and XylT2 genes. As summarized in Figure 12, Xyl_T04 TALEN™ plasmid DNAs induced very high frequencies of NHEJ mutations in both genes, ranging from 31.2% to 54.9%. In parallel, Xyl_T04 TALEN™ mRNAs also induced high frequencies of NHEJ mutations in both genes, ranging from 22.9% to 44.2%. Examples of TALEN™-induced mutations on XylT1 and XylT2 loci are shown in Figure 13.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### REFERENCES

Bashirullah A, Cooperstock R, Lipshitz H (2001) Spatial and temporal control of RNA stability. PNAS 98: 7025-7028.
Beurdeley M, Bietz F, Li J, Thomas S, Stoddard T, et al. (2013) Compact designer TALEN™ for efficient genome engineering. Nature Communications 4: 1762.
Bhushan S, Kuhn C, Berglund AK, Roth C, Glaser E (2006) The role of the N-terminal domain of chloroplast targeting peptides in organellar protein import and miss-sorting. FEBS Letters Volume 580, Issue 16, 10 July 2006, Pages 3966-3972.
Certo MT, Gwiazda KS, Kuhar R, Sather B, Curinga G, et al. (2012) Coupling endonucleases with DNA end-processing enzymes to drive gene disruption. Nature methods 9:973-975.Christou, P. (1997) Rice transformation: bombardment. Plant Mol Biol. 35 (1-2):197-203.
Chugh, A., Amundsen, E., Eudes, F. (2009) Translocation of cell-penetrating peptides and delivery of their cargoes in triticale microspores. Plant Cell Rep. 28 (5):801-10
Dormann, D., Madl, T., Valori, C.F., Bentmann, E., Tahirovic, S., Abou-Ajram, C., Kremmer, E., Ansorge, O., Mackenzie, I.R., Neumeann, M., Haass, C. (2012) Arginine methylation next to the PY-NLS modulates Transportin binding and nuclear import of FUS. EMBO J. 31(22):4258-75.
Frankel A.D., Pabo C.O. (1988) Cellular uptake of the tat protein from human immunodeficiency virus. Cell 55: 1189-1193.
Gaj T., Guo J., Kato Y., Sirk S., Barbas C. (2013) Targeted gene knockout by direct delivery of ZFN proteins. Nat Methods 9(8):805-807
Gasiunas, G., Barrangou, R., Horvath, P., Siksnys, V. (2012) Cas9-crRNA ribonucleoprotein complex mediates specific DNA cleavage for adaptive immunity in bacteria. PNAS 109(39):E2579-86.
Golds T., Maliga P., Koop H.U. (1993) Stable plastid transformation in PEG-treated protoplasts of Nicotiana tabacum. Nature Biotechnology 11:95-97.
Hicks, G.R., Raikhel, N.V. (1993) Specific binding of nuclear localization sequences to plant nuclei. Plant Cell 5(8):983-94
Klein T.M., Arentzen R., Lewis P.A., Fitzpatrick-McElligott S. (1992) Transformation of microbes, plants and animals by particle bombardment. Biotechnology (NY) 10(3):286-91.
Lakshmanan, M., Kodama, Y., Yoshizumi, T., Sudesh, K., Numata, K. (2013) Rapid and efficient delivery into plant cells using designed peptide carriers. Biomacromolecules 14(1):10-6
Laursen C.M., Krzyzek R.A., Flick C.E., Anderson P.C., Spencer T.M., (1994) Production of fertile transgenic maize by electroporation of suspension culture cells. Plant Mol Biol. 24(1):51-61.
Lloyd, D. Plaisier C.L., Carroll D, Drews G.N.,(2005) Targeted mutagenesis using zinc-finger nucleases in Arabidopsis. PNAS 102 :2232-2237.
Mäe, M., Langel, U. (2006) Cell-penetrating peptides as vectors for peptide, protein and oligonucleotide delivery. Curr. Opin. Pharmacol. 6(5):509-14.
Narsai R., Howell K., Millar A., O'Toole N., Small I., Whelan J. (2007) Genome-Wide Analysis of mRNA Decay Rates and Their Determinants in Arabidopsis thaliana. The Plant Cell 19:3418-3436.
Pacher M., Schmidt-Puchta W., Puchta H. (2007) Two unlinked double-strand breaks can induce reciprocal exchanges in plant genomes via homologous recombination and nonhomologous end joining. Genetics 175: 21-29.
Schwarze S.R., Ho A., Vocero-Akbani A., Dowdy S.F. (1999) In vivo protein transduction: delivery of a biologically active protein into the mouse. Science 285: 1569-1572.
Shevelev, I.V., Ramadanm K., Hübscher, U. (2002) The TREX2 3'-5' exonuclease physically interacts with DNA polymerase delta and increases its accuracy. ScientificWorldJournal 2:275-81.
Strasser R., Stadlmann J., Schahs M., Stiegler G., Quendler H., et al. (2008) Generation of glyco-engineered Nicotiana benthamiana for the production of monoclonal antibodies with a homogeneous human-like N-glycan structure. Plant biotechnology journal 6: 392-402.
Townsend J.A., Wright D.A., Winfrey R.J., Fu F., Maeder M.L., Joung J.K., Voytas D.F. (2009) High-frequency modification of plant genes using engineered zinc-finger nucleases. Nature 459: 442-445.
Wright D.A., Townsend J.A., Winfrey R.J., Jr., Irwin P.A., Rajagopal J., et al. (2005) High-frequency homologous recombination in plants mediated by zinc-finger nucleases. Plant J 44: 693-705.
Yoo S.D., Cho Y.H., Sheen J. (2007) Arabidopsis mesophyll protoplasts: a versatile cell system for transient gene expression analysis. Nature Protocols 2: 1565-1572.
Zhang Y., Zhang F., Li X, Baller J.A., Qi Y., et al. (2013) Transcription activator-like effector nucleases enable efficient plant genome engineering. Plant Physiology 161: 20-27.

## Claims

1. A method for targeted genetic modification of a plant genome without inserting exogenous genetic material into the genome comprising:
(i) providing a plant cell that comprises an endogenous gene to be modified;
(ii) providing a Cas9 endonuclease protein targeted to the endogenous gene; and
(iii) transfecting the plant cell with said Cas9 endonuclease protein using biolistic or protoplast transformation, such that the Cas9 endonuclease introduces one or more double stranded DNA breaks (DSB) in the genome, to produce a plant cell or cells having a detectable targeted genomic modification without the presence of any exogenous Cas9 genetic material in the plant genome.

2. The method of claim 1, wherein the Cas9 endonuclease protein is in the form of a purified protein.

3. The method of claim 1 or claim 2, wherein the Cas9 endonuclease protein further comprises one or more subcellular localization domains.

4. The method of claim 3, wherein the one or more subcellular localization domains comprise an SV40 nuclear localization signal, an acidic M9 domain of hnRNPA1, a PY-NLS motif signal, a mitochondrial targeting signal, or a chloroplast targeting signal.

5. The method of any one of claims 1 to 4, wherein the Cas9 endonuclease further comprises one or more cell penetrating peptide domains (CPPs).

6. The method of claim 5, wherein said one or more CPPs comprise a transactivating transcriptional activator (Tat) peptide, or a Pep-1 CPP domain.

7. The method of any one of claims 1 to 6, wherein the Cas9 endonuclease protein is co-transfected with one or more plasmids encoding one or more exonucleases comprising a member of the TREX exonuclease family, such as TREX2.

8. The method of any one of claims 1 to 7, wherein the endogenous gene to be modified is an acetolactate synthase gene, such as ALS1 or ALS2, or wherein the endogenous gene to be modified is a vacuolar invertase gene such as the potato (Solanum tuberosum) vacuolar invertase gene (VInv).

9. The method of any one of claims 1 to 8, wherein said plant cell is from a species of alfalfa, barley, bean, corn, cotton, flax, pea, rape, rice, rye, safflower, sorghum, soybean, sunflower, tobacco, or wheat.

10. The method of any one of claims 1 to 9, wherein transfection is effected through delivery of the Cas9 endonuclease protein into isolated plant protoplasts.

11. The method of any one of claims 1 to 9, wherein transfection is effected through delivery of the Cas9 endonuclease protein by biolistic transformation.

12. The method of claim 1, further comprising regenerating the plant cell or cells having the detectable targeted genomic modification into a plant.

13. A kit for the targeted genetic modification of a plant genome without inserting exogenous genetic material, said kit comprising:
(i) one or more Cas9 proteins;
(ii) one or more plant protoplasts or whole cultured plant cells;
and optionally
(iii) one or more DNA plasmid vectors encoding one or more TREX family exonucleases.

## Patentansprüche

1. Verfahren zur gezielten gentechnischen Veränderung eines Pflanzengenoms ohne Insertion von exogenem genetischem Material in das Genom, umfassend:
(i) Bereitstellen einer Pflanzenzelle, die ein zu modifizierendes endogenes Gen umfasst;
(ii) Bereitstellen eines Cas9-Endonuklease-Proteins, das auf das endogene Gen gerichtet wird; und
(iii) Transfizieren der Pflanzenzelle mit dem Cas9-Endonuklease-Protein unter Verwendung von biolistischer oder Protoplasten-Transformation, so dass die Cas9-Endonuklease einen oder mehrere Doppelstrang-DNA-Brüche (DSB) im Genom einführt, so dass eine Pflanzenzelle bzw. Pflanzenzellen mit einer nachweisbaren gezielten Modifikation erzeugt wird, ohne dass irgendein exogenes genetisches Cas9-Material im Pflanzengenom vorliegt.

2. Verfahren nach Anspruch 1, wobei das Cas9-Endonuklease-Protein in Form eines aufgereinigten Proteins vorliegt.

3. Verfahren nach Anspruch 1 oder Anspruch 2, wobei das Cas9-Endonuklease-Protein ferner eine oder mehrere Subzelluläre-Lokalisierung-Domänen umfasst.

4. Verfahren nach Anspruch 3, wobei die eine oder mehreren Subzelluläre-Lokalisierung-Domänen ein SV40-Kernlokalisierungssignal, eine saure M9-Domäne von hnRNPA1, ein PY-NLS-Motiv-Signal, ein Mitochondriales-Targeting-Signal oder ein Chloroplasten-Targeting-Signal umfassen.

5. Verfahren nach einem der Ansprüche 1 bis 4, wobei die Cas9-Endonuklease ferner eine oder mehrere zellpenetrierende Peptiddomänen (CPP) umfasst.

6. Verfahren nach Anspruch 5, wobei die eine oder mehreren CPP ein Transaktivierender-Transkriptionsaktivator(Tat)-Peptid oder eine Pep-1-CPP-Domäne umfassen.

7. Verfahren nach einem der Ansprüche 1 bis 6, wobei das Cas9-Endonuklease-Protein zusammen mit einem oder mehreren Plasmiden, die eine oder mehrere Exonukleasen codieren, die ein Mitglied der TREX-Exonuklease-Familie, wie z. B. TREX2, umfassen, transfiziert wird.

8. Verfahren nach einem der Ansprüche 1 bis 7, wobei es sich bei dem zu modifizierenden endogenen Gen um ein Acetolactat-Synthase-Gen, wie z. B. ALS1 oder ALS2, handelt oder wobei es sich bei dem zu modifizierenden endogenen Gen um ein VInv(Vacuolar Invertase)-Gen, wie z. B. das VInv(Vacuolar Invertase)-Gen aus Kartoffel (Solanum tuberosum), handelt.

9. Verfahren nach einem der Ansprüche 1 bis 8, wobei die Pflanzenzelle aus einer Spezies von Alfalfa, Gerste, Bohne, Mais, Baumwolle, Flachs, Erbse, Raps, Reis, Roggen, Färberdistel, Sorghum, Sojabohne, Sonnenblume, Tabak oder Weizen stammt.

10. Verfahren nach einem der Ansprüche 1 bis 9, wobei die Transfektion über Zuführung des Cas9-Endonuklease-Proteins in isolierte Pflanzenprotoplasten erfolgt.

11. Verfahren nach einem der Ansprüche 1 bis 9, wobei die Transfektion über Zuführung des Cas9-Endonuklease-Proteins durch biolistische Transformation erfolgt.

12. Verfahren nach Anspruch 1, ferner umfassend Regenerieren der Pflanzenzelle oder -zellen mit der nachweisbaren gezielten genomischen Modifikation in eine Pflanze.

13. Kit zur gezielten gentechnischen Veränderung eines Pflanzengenoms ohne Insertion von exogenem genetischem Material, wobei das Kit Folgendes umfasst:
(i) ein oder mehrere Cas9-Proteins;
(ii) einen oder mehrere Pflanzenprotoplasten bzw. eine oder mehrere intakte kultivierte Pflanzenzellen;
und gegebenenfalls
(iii) einen oder mehrere DNA-Plasmidvektoren, die eine oder mehrere Exonukleasen der TREX-Familie codieren.

## Revendications

1. Méthode de modification génétique ciblée d'un génome de plante sans insertion de matériel génétique exogène dans le génome, comprenant
(i) la fourniture d'une cellule végétale qui comprend un gène endogène à modifier ;
(ii) la fourniture d'une protéine de type endonucléase Cas9 ciblée vers le gène endogène ; et
(iii) la transfection de la cellule végétale par ladite protéine de type endonucléase Cas9 à l'aide d'une transformation biolistique ou protoplastique, de sorte que l'endonucléase Cas9 introduise une ou plusieurs cassures double brin de l'ADN (DSB) dans le génome, afin de produire une ou plusieurs cellules végétales ayant une modification génomique ciblée détectable sans la présence d'un quelconque matériel génétique de Cas9 exogène dans le génome de plante.

2. Méthode selon la revendication 1, dans laquelle la protéine de type endonucléase Cas9 se trouve sous la forme d'une protéine purifiée.

3. Méthode selon la revendication 1 ou la revendication 2, dans laquelle la protéine de type endonucléase Cas9 comprend en outre un ou plusieurs domaines de localisation subcellulaire.

4. Méthode selon la revendication 3, dans laquelle les un ou plusieurs domaines de localisation subcellulaire comprennent un signal de localisation nucléaire SV40, un domaine M9 acide de hnRNPA1, un signal de motif PY-NLS, un signal de ciblage mitochondrial, ou un signal de ciblage chloroplastique.

5. Méthode selon l'une quelconque des revendications 1 à 4, dans laquelle l'endonucléase Cas9 comprend en outre un ou plusieurs domaines de peptides de pénétration cellulaire (CPP).

6. Méthode selon la revendication 5, dans laquelle lesdits un ou plusieurs CPP comprennent un peptide activateur transactivateur transcriptionnel (Tat), ou un domaine de CPP Pep-1.

7. Méthode selon l'une quelconque des revendications 1 à 6, dans laquelle la protéine de type endonucléase Cas9 est cotransfectée avec un ou plusieurs plasmides codant pour une ou plusieurs exonucléases comprenant un membre de la famille des exonucléases TREX, tel que TREX2.

8. Méthode selon l'une quelconque des revendications 1 à 7, dans laquelle le gène endogène à modifier est un gène d'acétolactate synthase, tel qu'ALSl ou ALS2, ou où le gène endogène à modifier est un gène d'invertase vacuolaire tel que le gène d'invertase vacuolaire (VInv) de la pomme de terre (*Solanum tuberosum*)*.*

9. Méthode selon l'une quelconque des revendications 1 à 8, dans laquelle ladite cellule végétale appartient à une espèce de luzerne, d'orge, de haricot, de maïs, de coton, de lin, de pois, de colza, de riz, de seigle, de carthame, de sorgho, de soja, de tournesol, de tabac ou de blé.

10. Méthode selon l'une quelconque des revendications 1 à 9, dans laquelle la transfection est effectuée via la distribution de la protéine de type endonucléase Cas9 dans des protoplastes végétaux isolés.

11. Méthode selon l'une quelconque des revendications 1 à 9, dans laquelle la transfection est effectuée via la distribution de la protéine de type endonucléase Cas9 par transformation biolistique.

12. Méthode selon la revendication 1, comprenant en outre la régénération de la ou des cellules végétales possédant la modification génomique ciblée détectable dans une plante.

13. Kit de modification génomique ciblée d'un génome de plante sans insertion de matériel génétique exogène, ledit kit comprenant
(i) une ou plusieurs protéines de type Cas9 ;
(ii) un ou plusieurs protoplastes végétaux ou des cellules végétales cultivées entières ; et éventuellement
(iii) un ou plusieurs vecteurs de type plasmide d'ADN codant pour une ou plusieurs exonucléases de la famille TREX.
